# EUROPEAN PATENT SPECIFICATION

(11) **EP 0 464 153 B1**
(45) Date of publication and mention of the grant of the patent: **09.11.1994**
(21) Application number: 90907545.9
(22) Date of filing: 17.01.1990
(51) Int. Cl.: C07J 75/00, C07D 307/93, C07C 5/00

(54) **DEHALOGENATION OF ORGANIC COMPOUNDS USING TIN OR LEAD**
ENTHALOGENIERUNG ORGANISCHER VERBINDUNGEN UNTER VERWENDUNG VON ZINN ODER BLEI
DESHALOGENATION DE COMPOSES ORGANIQUES AU MOYEN D'ETAIN OU DE PLOMB

(30) Priority: 07.02.1989 US 307645
(43) Date of publication of application: 08.01.1992
(73) Proprietor: THE UPJOHN COMPANY, Kalamazoo, Michigan 49001 (US)
(72) Inventor: PEARLMAN, Bruce, Allen, Kalamazoo, MI 49008 (US)
(74) Representative: Perry, Robert Edward
(86) International application number: US9000157
(87) International publication number: WO9009394

(56) References cited:
- Chemical Abstracts, vol. 110, no. 21, 22 May 1989, Columbus, Ohio, US, see page 664, abstract 191900r, & JP, A, 63222122 (KANTO DENKA KOGYO CO., LTD) 16 Sept. 1988
- Chemical Abstracts, vol. 90, no. 7, 12 Febr. 1979, Colubus, Ohio, USm H.Parnes et al., see page 632, abstract 55156u
- Synthesis. no.7, July 1986, Georg Thieme Verlag, (Stuttgart, DE), A.Ono et al., pages 570-571, see the whole article
- The Journal of Organic Chemistry, vol. 25, no. 1, 29 Febr. 1960, American Chemical Society, C. Djerassi et al., pages 149-151
- Bulletin of the Chemical Society of Japan, vol. 62, no. 2, Febr. 1989, The Chemical Society of Japan, H. Tanaka et al., pages 627-629, see the whole article

## Description

### BACKGROUND OF THE INVENTION

### 1. Field of the Invention

The present invention is a process for the dehalogenation of 9α-halo steroids (I) to produce the corresponding 11-oxygenated steroids (II) which are known to be useful as pharmaceuticals. The process can also be used to dehalogenate other steroids and non-steroids to produce compounds which are useful in the production of pharmaceuticals.

### 2. Description of the Related Art

11-Oxygenated steroids, especially corticoids, are very important commercial steroids. These can generally be made by two methods; first by fermentation with a microorganism which will hydroxylate at the 11 position in the β configuration and second by starting with a Δ⁹⁽¹¹⁾- or Δ¹¹-steroid, forming the halohydrin and then dehalogenating. Processes for dehalogenation of 9α-halo-11-oxygenated steroids and 12α-halo-11-oxygenated steroids are known.

US Patent 3,480,622, Tetrahedron Letters 3151 (1964) and J. Am Chem. Soc., 88, 3016 (1966) describe a process for the debromination of a 9α-bromo-11-oxygenated steroid by reaction of the brominated steroid with several equivalents each of a chromium (II) salt and a hydrogen atom donor such as n-butane thiol. This process has several disadvantages. One is that it generates a Δ⁹⁽¹¹⁾-steroid and a 5,9-cyclosteroid as by-products, see US 3,480,622, column 3. The Δ⁹⁽¹¹⁾ compound produced is difficult to separate from the desired 11-oxygenated product (II). Another is that the chromium (II) salt and the chromium (III) salt by-product are toxic, so the process creates a toxic waste disposal problem. Another disadvantage is that the process requires a polar solvent such as DMF, DMSO and DMAC which are expensive.

US Patents 4,304,727 and 4,325,881 improved upon the process of US Patent 3,480,622 by using thioglycolic acid as the hydrogen atom donor.

US Patent application Serial No 88/02430 filed July 22, 1988 improves upon these processes by (1) adding the 9α-halo steroid to the chromium ion rather than adding the chromium ion to the 9α-halo steroid and (2) using < 1 equivalent of soluble chromium ion in the presence of a reagent capable of converting chromium (III) to chromium (II). This process offers significant advantages over the original process. However, it still generates chromium (III) as a waste product (although a lesser amount) and requires the use of expensive polar solvents.

It is known that the following reagents also dehalogenate alkyl halides: Raney nickel, palladium and hydrogen, platinum and hydrogen, zinc/acetic acid, cobalt phthalocyanine/sodium borohydride, lithium trimethoxyaluminum hydride/cuprous iodide, vanadium dichloride, lithium di-n-butyl-9-BBN and sodium cyanoborohydride/zinc chloride. However, none of the above agents which have been tried effectuated conversion of the 9α-halosteroids (I) to the corresponding 11-oxygenated steroids (II) cleanly. For example, treatment of 9α-bromohydrocortisone 21-acetate (I) with zinc/ethanol/water; palladium/carbon, hydrogen and Raney nickel/diox./ether each produce the Δ⁹⁽¹¹⁾ compound as the major product, see J. Am. Chem. Soc., 79, 1130 (1957). Treatment of 9α-bromoprednisolone 21-acetate (I) with chromous chloride/acetone produces a mixture of the Δ⁹⁽¹¹⁾ compound and the 5,9-cyclosteroid, see Proc. Chem. Soc. 207 (1961).

It has been known for some time that organotin hydrides such as tri-n-butyltin hydride dehalogenate 9α-halo steroids (I) to the corresponding 11-oxygenated steroids cleanly without formation of any Δ⁹⁽¹¹⁾ or 5,9-cyclosteroid by-product, see J. Org. Chem., 44, 151 (1979), footnote 25 in J. Am. Chem. Soc., 88, 3016 (1966) and US Patent 3,894,063. However, organotin hydrides are both expensive to prepare and are themselves toxic, see Kirk-Othmer Encyclopedia Chem. Tech. 23, 69 (1983). These disadvantages have precluded these reagents from being employed commercially.

The expense can be reduced by using a catalytic amount of tri-n-butyl tin chloride together with a stoichiometric amount of a cheap hydride donor (lithium aluminum hydride, sodium borohydride or sodium cyanoborohydride), see J. Org. Chem., 28, 265 (1963), J. Org. Chem., 40, 2554 (1975), J. Am. Chem. Soc., 108, 303 (1986) and J. Org. Chem., 52, 472 (1987). However, the problem of toxicity is not eliminated simply because a lesser amount of the organotin reagent is used. Also, the lithium aluminum hydride and sodium borohydride variants of this process are inoperable in the case of 11-oxygenated steroids (II) because these metal hydrides rapidly reduce the carbonyl group at C₃, C₂₀ and at C₁₁ (if present).

Another dehalogenation strategy that has been extensively investigated is that of using an organotin hydride that is attached to a polymer. Polymeric organotin hydrides have been described as useful for dehalogenating alkyl halides, see J. Org. Chem., 40, 1966 (1975), US Patent 3,975,334 and J. Chem. Soc. Chem. Comm., 798 (1984). These polymeric organotin hydrides can be easily separated from the desired 11-oxygenated steroid (II) products by filtration so their toxicity is irrelevant. However, these polymeric reagents are prepared by multi-step sequences and thus are too expensive to have commercial utility. In the case of the polymer described in J. Org. Chem., 40, 1966 (1975), an additional disadvantage is that regeneration of the hydride form from the halide form cannot be accomplished efficiently.

Inorganic tin(II) salts are both less expensive and less toxic than organotin hydrides (see Kirk-Othmer Encyclopedia Chem. Tech., 23 (1981) in particular pages 28 and 51). These salts are capable of effecting dehalogenation of certain "activated" alkyl halides (i.e., those in which the halogen is unusually susceptible to reduction) such as α-chloroacetophenone. The ease of dechlorination of α-chloroacetophenone is well documented; fro example, see J. Am. Chem. Soc., 85, 2768 (1963). The following are examples of inorganic tin reagents that dechlorinate α-chloroacetophenone: stannous chloride/diisobutylaluminum hydride [Chem. Lett. 2069 (1984)], stannous chloride/sodium borohydride [Z. Naturforsch. B., 416, 1568 (19860], stannous chloride/sodium bromide [Synthesis, 570 (1986)], stannous chloride/sodium thiocyanate [Synthesis, 406 (1987)] and stannous chloride/sodium hydrosulfide [Syn. Communications, 16, 653 (1986)]. However, inorganic tin(II) salts do not dehalogenate simple, unactivated alkyl halides such as n-octyl bromide or chloride: see J. Organometallic Chem., 97, 167 (1975), where it is stated that "... reactions of stannous bromide or chloride with alkyl bromides or chlorides do not proceed." None of the above-listed inorganic tin (II) salts dehalogenate 9α-halosteroids (I).

Other functional groups that inorganic tin(II) salts are capable of reducing include organic azides (by stannous chloride), see Tetrahedron Lett., 28, 5941 (1987) and nitro compounds (by stannous chloride/hydrochloric acid), see Org. Synthesis Coll. Vol. II, 130 (1943). However, the fact that these functional groups are highly susceptible to reduction is well documented. For example, even ammonium sulfide and sodium hydrosulfide, which do not reduce unactivated alkyl halides, reduce organic asides and nitro compounds at room temperature, see Tetrahedron Lett., 2031, (1974) for azides and Org. Reactions, 20, 455 (1973) for nitro compounds. Thus, the utility of inorganic tin (II) salts is limited by their low reducing power to reduction of easily reducible functional groups.

Changing the ligands on the inorganic tin(II) salts from chlorine to alklythio in order to enhance its reactivity is known. For example, see Tetrahedron Lett., 28, 5941 (1987), where it is reported that triethylammonium tris(phenylthio)stannite [(C₂H₅)₃N⁺ SN(Sφ)₃⁻] is a more powerful reducing agent than is stannous chloride toward organic azides. However, even this reagent is not useful for dehalogenation of 9α-halosteroids (I) or other unactivated alkyl halides. Thus simply increasing the reducing power of the inorganic tin(II) salts alone does not solve the problem of dehalogenation of unactivated alkyl halides.

### SUMMARY OF INVENTION

A novel process according to the present invention comprises the dehalogenation of a halogenated compound of any of formulae I, III, V, VII, IX, XIII, XV, XVII, XIX and XXI, to give a compound of formula II, IV, VI, VIII, X, XII, XIV, XVI, XVIII, XX or XXII, respectively. The various groups etc. in these formulae are defined in claims 1 and 7. The reaction, which is capable of reducing relatively unreactive functional groups, such as unreactivated alkyl halides, comprises contacting the halogenated compound with a metal selected from the group consisting of tin (0), a tin (II) salt, lead (0) or a lead (II) salt, a hydrogen atom donor and an initiator.

### DETAILED DESCRIPTION OF THE INVENTION

The present invention is a process for the preparation of 11-oxygenated steroids of formula (II) which comprises contacting 9α-halo steroids of formula (I) with (1) tin (0), a tin (II) salt, lead (0) or a lead (II) salt, (2) a hydrogen atom donor and (3) an initiator. It is preferred that a weak base also be present during the contacting.

The 9α-halo steroidal (I) starting materials are either known to those skilled in the art or can readily be prepared from known compounds by means known to those skilled in the art.

The tin (0) or lead (0) are added as a finely divided powder; tin (0) is preferred. Tin (II) and lead (II) salts are also operable; virtually any such salt is operable. For example, preferred stannous salts include, fluoride, chloride, bromide, iodide, oxalate, oxide, sulfide, methoxide, ethoxide, phenoxide, catecholate, ethylene glyoxide, 1,3-propylene glyoxide, acetate, propionate, 2-ethylhexanoate, pyrophosphate, sulfate, tetrafluoroborate as well as dimethyl stannane and dicyclopentandienyl stannane. More preferred stannous salts are chloride, 2-ethylhexanoate and ethylene glyoxide. Examples of preferred lead (II) salts include fluoride, chloride, bromide, iodide, oxide, acetate, carbonate, sulfate, perchlorate, thiocyanate, 2-ethylhexanoate, methylmercaptide, orthosilicate, orthophosphate, 2,4-pentanedionate, sulfide, stannate, tetrafluoroborate, tungstate, zirconate, trifluoroacetate and titanate. More preferred are 2-ethylhexanoate, acetate and chloride.

If less than two equivalents of tin (0) or lead (0) are used, the dehalogenation is incomplete. The amount required for reaction is about 2 equivalents in the case of 325 mesh tin (0) powder. With other forms of tin metal such as foil, wire, shot, stick, bar, granulation and mossy tin the prosess is operable but a larger excess is required because their surface areas are smaller. The 325 mesh tin powder is the preferred metal (0). The amount of tin (II) or lead (II) salt needed for the reaction to go to completion is about 2 equivalents. With less the reaction produces dehalogenated product but does not go to completion. Use of greater than about two equivalents of tin (II) or lead (II) salt is wasteful but not deleterious.

The hydrogen atom donor is any compound which is capable of donating a hydrogen atom to the halogen-bearing carbon atom of the 9α-halo steroid (I). Preferably, the hydrogen atom donor is any compound which, when contacted with 9α-bromoprednisolone 21-acetate (I) and tin (0) in sec-butanol for 1 hour at 75 to 80°C, produces prednisolone 21-acetate (II) in the same as or a higher yield that that which is obtained using hypophosphorous acid, a known hydrogen atom donor. Suitable hydrogen atom donors include hydrogen bromide,
hydrogen iodide,
hypophosphorous acid,
1,2- and 1,4-dihydrobenzene,
1,2 and 1,4-dihydrotoluene,
1,2- and 1,4-dihydro-(ortho, meta, or para)-xylene,
1,4-dihydronaphthalene,
9,10-dihydroanthracene,
cyclopentadiene,
1-benzyl-1,4-dihydronicotinamide,
3,5-di-t-butyl-4-hydroxytoluene,
H-Si-(X₂)₃, H-Sn-(X₂)₃, H-Ge-(X₂)₃, H-P-(X₂)₂, H-Se-X₂, H-B-(X₂)₃ or H-S-X₂ where, when more than one X₂ is present the X₂'s are the same or different and are -H,
C₁-C₁₀ alkyl,
C₅-C₁₀ cycloalkyl,
α-napththyl,
β-naphthyl,
-φ optionally substituted with 1 or 2 X₃, where X₃ is
-F,
-Cl,
-Br,
-I,
-φ,
C₁-C₅ alkyl,
C₅-C₇ cycloalkyl,
-OX₄ where X₄ is -H, C₁-C₅ alkyl or C₅-C₇ cycloalkyl,
-COOX₄ where X₄ is as defined above,
-N(X₅)₂ where the X₅'s are the same or different and are -H, C₁-C₅, and where the X₅'s are taken together with the attached nitrogen atom and optionally another heteroatom, to form a heterocyclic ring selected from the group consisting of pyrrolidine, piperidine, morpholine, piperazine or N-methylpiperazine,
3-mercaptopropionic acid,
mercaptoacetic acid,
2-mercaptopropionic acid,
ethane dithiol,
propane 1,3-dithiol. The preferred hydrogen atom donors are H-S-X₂.

The amount of the hydrogen atom donor required is from about 2 to about 10 equivalents, preferrably about 2 to about 5 equivalents, more preferrably about 2 to about 4 equivalents. If less than two equivalents are used, dehalogenated product is produced but undesireable side reactions occur. In the case of 3-mercaptopropionic acid, tin (0) and 9α-bromoprednisolone 21-acetate, 2-4 equivalents are required to suppress these side reactions. Initiators are selected from the group consisting of AIBN, oxygen, triethylborane optionally combined with oxygen, 1,1'-azobis(cyclohexane carbonitrile), dibenzoylperoxide, lauroyl peroxide, succinic acid peroxide, di-t-butylperoxyoxalate, di(2-ethylhexyl)peroxydicarbonate, t-butylperbenzoate, t-amylperoxypivalate, di-t-butylperoxide, dicumylperoxide, light of about 250 to about 350 mµ optionally combined with benzophenone, t-butylhydroperoxide optionally combined with acetic acid, hydrogen peroxide combined with ferrous perchlorate, t-butylperbenzoate combined with cuprous bromide, chloride or iodide, methylethylketone peroxide combined with cobalt naphthenate or cobalt octoate, potassium persulfate and potassium nitrosodisulfonate.

Air can fulfill the function of an initiator. For example, it has been found that when argon was bubbled through a reaction mixture containing a 9α-bromoprednisolone 21-acetate (I), 6.03 equivalents of tin (0) powder, and 10.02 equivalents of 3-mercaptopropionic acid in THF to purge all traces of dissolved oxygen, only about 15% of the 9α-bromoprednisolone 21-acetate (I) had been debrominated after 63 hr at 20-25°. When the reaction mixture was then exposed to air, conversion to prednisolone 21-acetate (II) speeded up dramatically and was complete within 45 hr. Air is not a preferred initiator because it oxidizes the tin (0) and thiol (if present) competitively. Preferred initiators are AIBN and dibenzoylperoxide; most preferred is AIBN.

The amount of initiator is not critical. The process operates with 0.1 mole % or lower, but is slow. Other factors being equal, the greater the amount of initiator, the faster the reaction rate. The amount that results in the most convenient rate is about 1 to about 10 mole %.

It is preferred to include in the reaction mixture approximately one molar equivalent of a weak base. A weak base is a base whose conjugate acid has a pKₐ of about 2 to about 12. Suitable weak bases include compounds selected from the group consisting of
3-mercaptopropionate;
(X₆)₃-N where the X₆'s are the same or different and are
-H,
C₁-C₁₀ alkyl,
C₅-C₁₀ cycloalkyl,
α-naphthyl,
β-naphthyl,
-φ optionally substituted with 1 or 2 X₇, where X₇ is
-F,
-Cl,
-Br,
-I,
-φ,
C₁-C₅ alkyl,
C₅-C₇ cycloalkyl,
-OX₈ where X₈ is -H, C₁-C₅ alkyl or C₅-C₇ cycloalkyl,
-COOX₈ where X₈ is as defined above,
-N(X₉)₂ where the X₉'s are the same or different and are -H, C₁-C₅, and where the X₉'s are taken together with the attached nitrogen atom and optionally another heteroatom, to form a heterocyclic ring selected from the group consisting of pyrrolidine, piperidine, morpholine, piperazine or N-methylpiperazine,
X₆-COO⁻ where X₆ is as defined above;
citrate;
oxalate;
tartrate;
imidazole;
N-methylimidazole;
2-methylimidazole;
pyridine;
4-dimethylaminopyridine;
(any isomer of) lutidine;
collidine,
N,N'-dimethylpiperazine;
1,4-diazabicyclo[2.2.2]octane;
1,8-diazabicyclo[5.4.0]undec-7-ene or
1,5-diazabicyclo[4.3.0]non-5-ene.
Without the weak base the reaction generates minor amounts of less polar impurities, which makes purification of the final product more difficult. A strong base can also be used if the hydrogen atom donor is 3-mercaptopropionic acid, since the strong base will convert the 3-mercaptopropionic acid to 3-mercaptopripionate salt, which serves as the weak base, see, for example, EXAMPLE 1 in which potassium tertiary butoxide is used.

The preferred reaction temperature is determined by the particular initiator used. Acceptable and preferred temperature ranges for various initiators are set forth in TABLE 1, see CHART C.

The reaction is performed either neat (in which case the hydrogen atom donor is the solvent) or in an inert solvent. Suitable inert solvents include water, alcohols, carboxylic acids, substituted amides, aliphatic or aromatic hydrocarbons, halogenated hydrocarbons, cyclic ethers, esters, ketones, nitriles and mixtures thereof. The 9α-halo steroids (I) need not even be soluble for the reaction to take place. In fact, superior yields are obtained in solvents in which the 9α-halo steroid (I) is not soluble. Thus the more preferred solvents are 1,2-dichloroethane, sec-butanol and isopropanol. Even more preferred are sec-butanol and isopropanol because they are water-miscible so that the 11-oxygenated steroid (II) product can be readily obtained by water knock-out. The preferred amount of solvent is 2-8 ml/g of 9α-halo steroid (I), although the process operates efficiently with greater or lesser amounts.

The 9α-halo steroid (I), metal (0) or metal (II) salts, hydrogen atom donor, initiator, weak base and solvent can be mixed in any order. After mixing, the temperature is raised to the temperature at which the reaction occurs. While the reaction is occuring the mixture is stirred or agitated vigorously so that the metal (0) or metal (II) salt is dispersed evenly throughout the reaction mixture.

When the reaction is complete, the 11-oxygenated steroids (II) are isolated and purified by known means.

Besides being useful for the conversion of the 9α-halo steroids (I) to the corresponding 11-oxygenated steroids (II), the process of the present invention is also useful in the same way for the conversion of the particular halogenated compounds of formulas (III, V, VII, IX, XI, XIII, XV, XVII, XIX and XXI) to the corresponding dehalogenated compounds of formulas (IV, VI, VIII, X, XII, XIV, XVI, XVIII, XX and XXII). The information regarding how to perform the process of the present invention with the 9α-halo steroids (I) is generally the same, with non-critical variations, for the halogenated compounds of formulas (III, V, VII, IX, XI, XIII, XV and XVII).

The product 11-oxygenated steroids (II) and the dehalogenated compounds are either useful pharmaceutical agents or intermediates in the preparation of useful pharmaceutical agents.

### DEFINITIONS AND CONVENTIONS

The definitions and explanations below are for the terms as used throughout this entire document including both the specification and the claims.

### I. CONVENTIONS FOR FORMULAS AND DEFINITIONS OF VARIABLES

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" where "i" is an integer. These variable substituents are either monovalent or bivalent, that is, they represent a group attached to the formula by one or two chemical bonds. For example, a group Z₁ would represent a bivalent variable if attached to the formula CH₃-C(=Z₁)H. Groups Rᵢ and Rⱼ would represent monovalent variable substituents if attached to the formula CH₃-CH₂-C(Rᵢ)(Rⱼ)H₂. When chemical formulas are drawn in a linear fashion, such as those above, variable substituents contained in parentheses are bonded to the atom immediately to the left of the variable substituent enclosed in parenthesis. When two or more consecutive variable substituents are enclosed in parentheses, each of the consecutive variable substituents is bonded to the immediately preceding atom to the left which is not enclosed in parentheses. Thus, in the formula above, both Rᵢ and Rⱼ are bonded to the preceding carbon atom. Also, for any molecule with an established system of carbon atom numbering, such as steroids, these carbon atoms are designated as Cᵢ, where "i" is the integer corresponding to the carbon atom number. For example, C₆ represents the 6 position or carbon atom number in the steroid nucleus as traditionally designated by those skilled in the art of steroid chemistry. Likewise the term "R₆" represents a variable substituent (either monovalent or bivalent) at the C₆ position.

Chemical formulas or portions thereof drawn in a linear fashion represent atoms in a linear chain. The symbol "-" in general represents a bond between two atoms in the chain. Thus CH₃-0-CH₂-CH(Rᵢ)-CH₃ represents a 2-substituted-1-methoxypropane compound. In a similar fashion, the symbol "=" represents a double bond, e.g., CH₂=C(Rᵢ)-O-CH₃, and the symbol "≡" represents a triple bond, e.g., HC≡C-CH(Rᵢ)-CH₂-CH₃. Carbonyl groups are represented in either one of two ways: -CO- or -C(=O)-, with the former being preferred for simplicity.

Chemical formulas of cyclic (ring) compounds or molecular fragments can be represented in a linear fashion. Thus, the compound 4-chloro-2-methylpyridine can be represented in linear fashion by N*=C(CH₃)-CH=CCl-CH=C*H with the convention that the atoms marked with an asterisk (*) are bonded to each other resulting in the formation of a ring. Likewise, the cyclic molecular fragment, 4-(ethyl)-1-piperazinyl can be represented by -N*-(CH₂)₂-N(C₂H₅)-CH₂-C*H₂.

A rigid cyclic (ring) structure for any compounds herein defines an orientation with respect to the plane of the ring for substituents attached to each carbon atom of the rigid cyclic compound. For saturated compounds which have two substituents attached to a carbon atom which is part of a cyclic system, -C(X₁)(X₂)- the two substituents may be in either an axial or equatorial position relative to the ring and may change between axial/equatorial. However, the position of the two substituents relative to the ring and each other remains fixed. While either substituent at times may lie in the plane of the ring (equatorial) rather than above or below the plane (axial), one substituent is always above the other. In formulas depicting such compounds, a substituent (X₁) which is "below" another substituent (X₂) will be identified as being in the alpha (α) configuration and is identified by a broken, dashed or dotted line attachment to the carbon atom, i.e., by the symbol "- - -" or "...". The corresponding substituent attached "above" (X₂) the other (X₁) is identified as being in the beta (β) configuration and is indicated by an unbroken line attachment to the carbon atom.

When a variable substituent is bivalent, the valences may be taken together or separately or both in the definition of the variable. For example, a variable Rᵢ attached to a carbon atom as -C(=Rᵢ)- might be bivalent and be defined as oxo or keto (thus forming a carbonyl group (-CO-) or as two separately attached monovalent variable substituents α-Rᵢ₋ⱼ and β-Rᵢ₋ₖ. When a bivalent variable, Rᵢ, is defined to consist of two monovalent variable substituents, the convention used to define the bivalent variable is of the form "α-Rᵢ₋ⱼ:β-Rᵢ₋ₖ" or some variant thereof. In such a case both α-Rᵢ₋ⱼ and β-Rᵢ₋ₖ are attached to the carbon atom to give -C(α-Rᵢ₋ⱼ)(β-Rᵢ₋ₖ)-. For example, when the bivalent variable R₆, -C(=R₆)- is defined to consist of two monovalent variable substituents, two monovalent variable substituents are α-R₆₋₁:β-R₆₋₂, .... α-R₆₋₉:β-R₆₋₁₀, etc, giving -C(α-R₆₋₁)(β-R₆₋₂)-, .... -C(α-R₆₋₉) (β-R₆₋₁₀)-, etc. Likewise, for the bivalent variable R₁₁, -C(=R₁₁)-, two monovalent variable substituents are α-R₁₁₋₁:β-R₁₁₋₂. For a ring substituent for which separate α and β orientations do not exist (e.g. due to the presence of a carbon carbon double bond in the ring), and for a substituent bonded to a carbon atom which is not part of a ring the above convention is still used, but the α and β designations are omitted.

Just as a bivalent variable may be defined as two separate monovalent variable substituents, two separate monovalent variable substituents may be defined to be taken together to form a bivalent variable. For example, in the formula -C₁(Rᵢ)H-C₂(Rⱼ)H- (C₁ and C₂ efine arbitrarily a first and second carbon atom, respectively) Rᵢ and Rⱼ may be defined to be taken together to form (1) a second bond between C₁ and C₂ or (2) a bivalent group such as oxa (-O-) and the formula thereby describes an epoxide. When Rᵢ and Rⱼ are taken together to form a more complex entity, such as the group -X-Y-, then the orientation of the entity is such that C₁ in the above formula is bonded to X and C₂ is bonded to Y. Thus, by convention the designation "... Rᵢ and Rⱼ are taken together to form -CH₂-CH₂-O-CO- ..." means a lactone in which the carbonyl is bonded to C₂. However, when designated "... Rⱼ and Rᵢ are taken together to form -CH₂-CH₂-O-CO-the convention means a lactone in which the carbonyl is bonded to C₁.

The carbon atom content of variable substituents is indicated in one of two ways. The first method uses a prefix to the entire name of the variable such as "C₁-C₄", where both "1" and "4" are integers representing the minimum and maximum number of carbon atoms in the variable. The prefix is separated from the variable by a space. For example, "C₁-C₄ alkyl" represents alkyl of 1 through 4 carbon atoms, (including isomeric forms thereof unless an express indication to the contrary is given). Whenever this single prefix is given, the prefix indicates the entire carbon atom content of the variable being defined. Thus C₂-C₄ alkoxycarbonyl describes a group CH₃-(CH₂)ₙ-0-CO- where n is zero, one or 2. By the second method the carbon atom content of only each portion of the definition is indicated separately by enclosing the "Cᵢ-Cⱼ" designation in parentheses and placing it immediately (no intervening space) before the portion of the definition being defined. By this optional convention (C₁-C₃) alkoxycarbonyl has the same meaning as C₂-C₄ alkoxycarbonyl because the "C₁-C₃" refers only to the carbon atom content of the alkoxy group. Similarly while both C₂-C₆ alkoxyalkyl and (C₁-C₃)alkoxy(C₁-C₃)alkyl define alkoxyalkyl groups containing from 2 to 6 carbon atoms, the two definitions differ since the former definition allows either the alkoxy or alkyl portion alone to contain 4 or 5 carbon atoms while the latter definition limits either of these groups to 3 carbon atoms.

### II. DEFINITIONS

All temperatures are in degrees Centigrade.

TLC refers to thin-layer chromatography.

THF refers to tetrahydrofuran.

DMF refers to dimethylformamide.

DMSO refers to dimethysulfoxide.

DMAC refers to dimethylacetamide.

AIBN refers to 1,1'-azobis[isobutyronitrile].

φ refers to phenyl (C₆H₅).

When solvent pairs are used, the ratios of solvents used are volume/volume (v/v).

Hydrocortisone refers to 11β,17α,21-trihydroxypregn-4-ene-3,20-dione.

Prednisolone refers to 11β,17α,21-trihydroxypregna-1,4-diene-3,20-dione.

∼ indicates that there are 2 possible orientations for the attached group, (1) α or β when attached to the steroid ring and (2) cis or trans when attached to a carbon atom of a double bond.

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### PREPARATION 1 3α-Iodo-5α-cholestane (XIX)

Following the general procedure described in J. Chem. Soc. Perkin I. 2866 (1980) a solution of dihydrocholesterol (5.0000 g), imidazole (2.6272 g) and triphenylphosphine (6.7481 g) in toluene (124 ml) is heated to reflux, then treated with iodine (9.7970 g). After 75 min, conversion to 3α-iodo-5α-cholestane is complete as measured by TLC (ethyl acetate/heptane, 1/1). The reaction mixture is then cooled to 20-25°, washed with saturated aqueous sodium bicarbonate (3 x 50 ml) followed by aqueous sodium thiosulfate (15%, 2 x 50 ml), followed by water (50 ml). The toluene phase is then dried over magnisium sulfate and concentrated to a solid. The solid is chromatographed on silica gel (330 g) eluting with ethyl acetate/hexane (1/99). The approprate fractions are pooled and concentrated to give the title compound.

### EXAMPLE 1 Debromination of 9α-Bromoprednisolone 21-Acetate (I) to Prednisolone 21-Acetate (II) Using Tin Powder

A solution of potassium tertiary butoxide in THF (20%, 10.0979 g) is added dropwise to a solution of 3-mercaptopropionic acid (6.3849 g) in secondary butanol (30 ml). The resulting mixture is treated with tin powder (325 mesh, 7.1251 g) followed by 9α-bromoprednisolone 21-acetate [I, JACS 77, 4438 (1955), 7.2211 g) followed by AIBN (0.4944 g). Secondary butanol (30 ml) is used for a rinse. The resulting slurry is heated at 82° under an atmosphere of nitrogen with vigorous stirring until TLC (acetone/methylene chloride/cyclohexane; 2/5/3) indicates that conversion of the 9α-bromoprednisolone 21-acetate to prednisolone 21-acetate is complete (8 hr). After 30 min the reaction mixture is added dropwise to water (600 ml) at 0°. The resulting mixture is stirred at 0° for 25 min. The solids are filtered off and dried by a stream of air. The solids are stirred in methylene chloride/methanol (1/1, 2 x 75 ml) and filtered. The filtrate is concentrated under reduced pressure to give a solid which is prednisolone 21-acetate in essentially pure form as measured by TLC.

The prednisolone 21-acetate is taken up in methylene chloride/methanol (1/1, 54 ml) with a brief warming over a steam bath. This mixture is treated with magnesol (0.503 g) and activated charcoal (0.501 g), stirred at 30° for 15 min, filtered thru celite and the filter cake washed with methylene chloride/methanol (1/1, 5 x 5 ml). The filtrate is then concentrated by distillation thru a short-path (bath temperature is 65°) to a volume of 18 ml. The resulting slurry is then diluted with ethyl acetate (36 ml) and reconcentrated to 18 ml. Again the mixture is diluted with ethyl acetate (36 ml) and reconcentrated to 18 ml. The slurry is diluted with ethyl acetate (17 ml) and water (1 ml), reconcentrated to 18 ml, cooled to -20° for 15 min and filtered. The filter cake is washed with -20° ethyl acetate (3 x 5 ml). The cake is then dried by a stream of nitrogen to give prednisolone 21-acetate.

### EXAMPLE 2 Debromination of 9α-Bromohydrocortisone 21-Acetate (I) to Hydrocortisone 21-Acetate (II) Using Tin Powder

3-Mercaptopropionic acid (2.1312 g) is treated with solid sodium bicarbonate (0.4215 g) and stirred at 20-25° for 30 min. 1,2-Dichloroethane (20 ml) is then added, followed by tin powder (325 mesh, 1.1907 g), 9α-bromohydrocortisone 21-acetate [I, JACS 77, 4438 (1955), 2.4170 g] and AIBN (0.0828 g). The resulting slurry is is heated at 80° under an atmosphere of nitrogen until TLC analysis (acetone/methylene chloride/cyclohexane; 2/5/3) indicates that conversion to hydrocortisone 21-acetate is complete (4 hr). The reaction mixture is diluted with acetic acid in 1 ml portions until all the precipitated solids dissolve (5 ml). Next activated charcoal (0.204 g) is added and the mixture filtered thru celite. The filtrate is then treated with a solution of potassium carbonate (89.1 mmoles) and potassium bicarbonate (49.9 mmoles) in water (42 ml). The slurry is then stirred at 0° for 1 hr and filtered. The cake is washed with water (2 x 20 ml) followed by methylene chloride (10 ml). The solids are then air-dried to give hydrocortisone 21-acetate (II).

### EXAMPLE 3 Debromination of 9α-Bromoprednisolone 21-Acetate (I) to Prednisolone 21-Acetate (II) Using Stannous 2-Ethylhexanoate

A mixture of stannous 2-ethylhexanoate (4.0728 in THF (10 ml) is treated with dropwise with a mixture of 3-mercaptopropionic acid (3.2043 g) in THF (10 ml). The resulting slurry is then treated with 9α-bromoprednisolone 21-acetate (I, 2.4083 g) followed by AIBN (0.1644 g). The mixture is stirred vigourously at reflux under an atmosphere of argon for 100 min at which time conversion to prednisolone 21-acetate (II) is complete as measured by TLC.

### EXAMPLE 4 Debromination of 9α-Bromoprednisolone 21-Acetate (I) to Prednisolone 21-Acetate (II) Using Tin Powder

A mixture of potassium acetate (0.5903 g) in neat 3-mercaptopropionic acid (1.5921 g) is treated successively with 9α-bromoprednisolone 21-acetate (I, 2.4080 g), tin powder (325 mesh, 1.7828 g), AIBN (0.1650 g) and isopropanol (5.0 ml). The resulting slurry is stirred vigorously at reflux under an argon atomosphere for 1.5 hr at which time conversion to prednisolone 21-acetate (II) is complete as measured by TLC. The reaction mixture is then poured into water (0°, 125 ml) using isopropanol (6.0 ml) for rinsing the reaction flask. After stirring for 1 hr at 0°, the solids which consist of a mixture of prednisolone 21-acetate and tin powder are filtered off and dried by a nitrogen stream. The prednisolone 21-acetate is purified as in EXAMPLE 1.

### EXAMPLE 5 Debromination of 9α-Bromoprednisolone 21-Acetate (I) to Prednisolone 21-Acetate (II) Using Lead Powder

A mixture of lead powder (325 mesh, 0.2202 g), 3-mercaptopropionic acid (0.5428 g) and 9α-bromoprednisolone 21-acetate (I, 0.0825 g) in THF (3 ml) is refluxed under an atmosphere of nitrogen. After 38 hr conversion to prednisolone 21-acetate (II) is complete. The product is isolated and purified as in EXAMPLE 1.

### EXAMPLE 6 Deiodination of 3α-Iodo-5α-cholestane (XIX) to 5α-Cholestane (XX)

A mixture of 3α-iodo-5α-cholestane (XIX, PREPARATION 1, 0.4994 g), 3-mercaptopropionic acid (0.9697 g) and AIBN (0.0343 g) in THF (3.0 ml) is treated with stannous ethyleneglyoxide (0.5370 g). The resulting slurry is stirred vigorously at reflux under an argon atmosphere for 15.5 hr. Additional stannous ethyleneglycoxide (0.3583 g), 3-mercaptopropionic acid (0.6435 g) and AIBN (0.0428 g) is added. The mixture is refluxed an additional 6 hr at which time the TLC indicates that conversion to 5α-cholestane is complete. The mixture is poured into aqueous hydrochloric acid (5%, 50 ml) and extracted with cyclohexane (2 x 20 ml). The cyclohexane extracts are washed with a solution of sodium hydroxide (10%) and sodium sulfite (5%) in water and filtered thru celite. The cyclohexane phase is dried over magnesium sulfate and concentrated under reduced pressure to give 5α-cholestane which is identical with an authentic sample of 5α-cholestane by NMR, CMR and TLC (hexane, R_{f} = 0.85).

### EXAMPLE 7 Deiodination of 1-Iodo-n-undecane (XXI) to n-Undecane (XXII)

A mixture of 1-iodo-n-undecane (XXI, 1.403 g), 3-mercaptopropionic acid (1.596 g) and AIBN (0.1641 g) in isopropanol (4.0 ml) is treated with tin powder (325 mesh, 1.7792 g). The resulting slurry is then stirred vigorously at reflux under an argon atmosphere for 4.5 hr. Additional tin powder (1.7597 g), 3-mercaptopropionic acid (1.596 g) and AIBN (0.1644 g) is then added and the resulting mixture is refluxed for another 1.5 hr at which time conversion to n-undecane (XXII) is complete. The mixture is poured into half saturated ammonium chloride (10 ml) and extracted with pentane. The pentane phase is washed with sodium thiosulfate (5%, 10 ml) followed by aqueous sodium bicarbonate (5%, 10 ml). The pentane phase is then washed with aqueous hydrochloric acid (10%, 10 ml), followed by a solution of sodium thiosulfate (5%) and sodium bicarbonate (5%) in water (10 ml), followed by water (10 ml). The pentane phase is dried over magnesium sulfate and concentrated under reduced pressure to give an oil and solids. The solids are filtered off and the filtrate is concentrated under reduced pressure to give an oil which is identical with an authentic sample of n-undecane by NMR, CMR and TLC (hexane, R_{f} = 0.75).

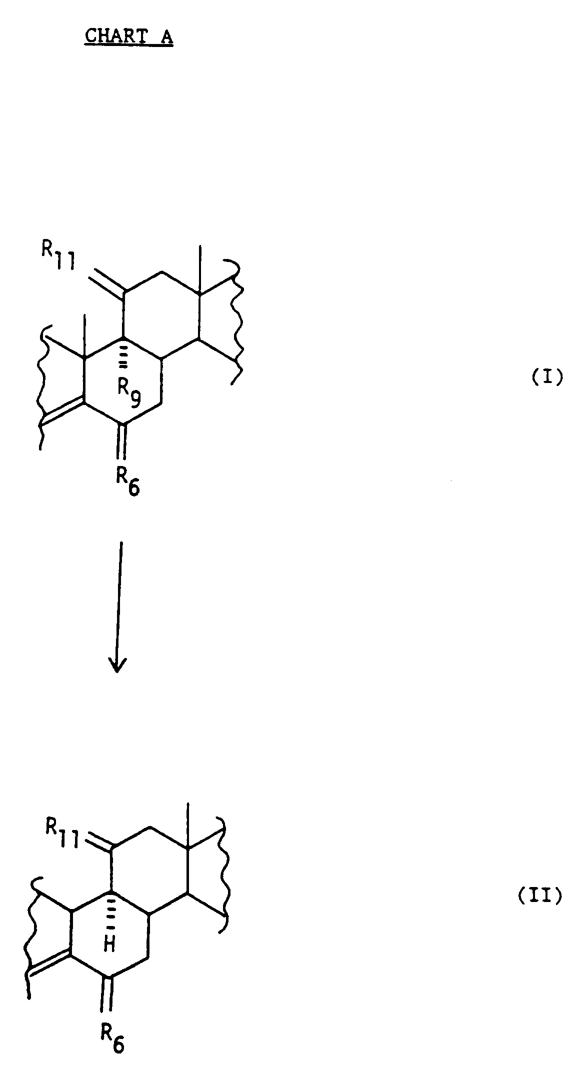

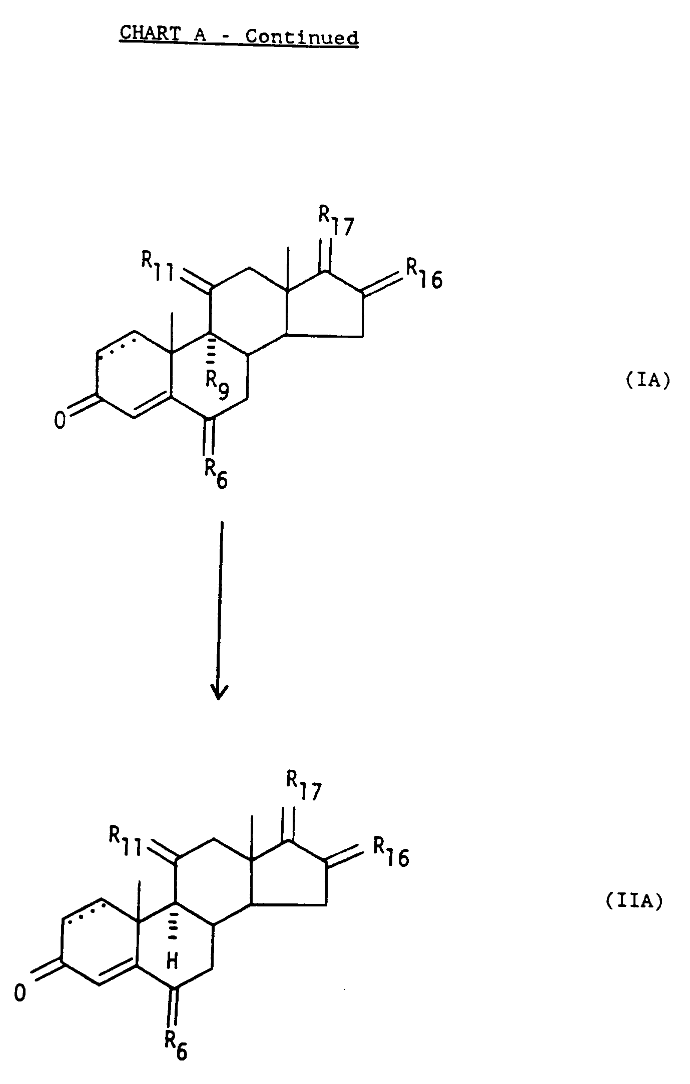

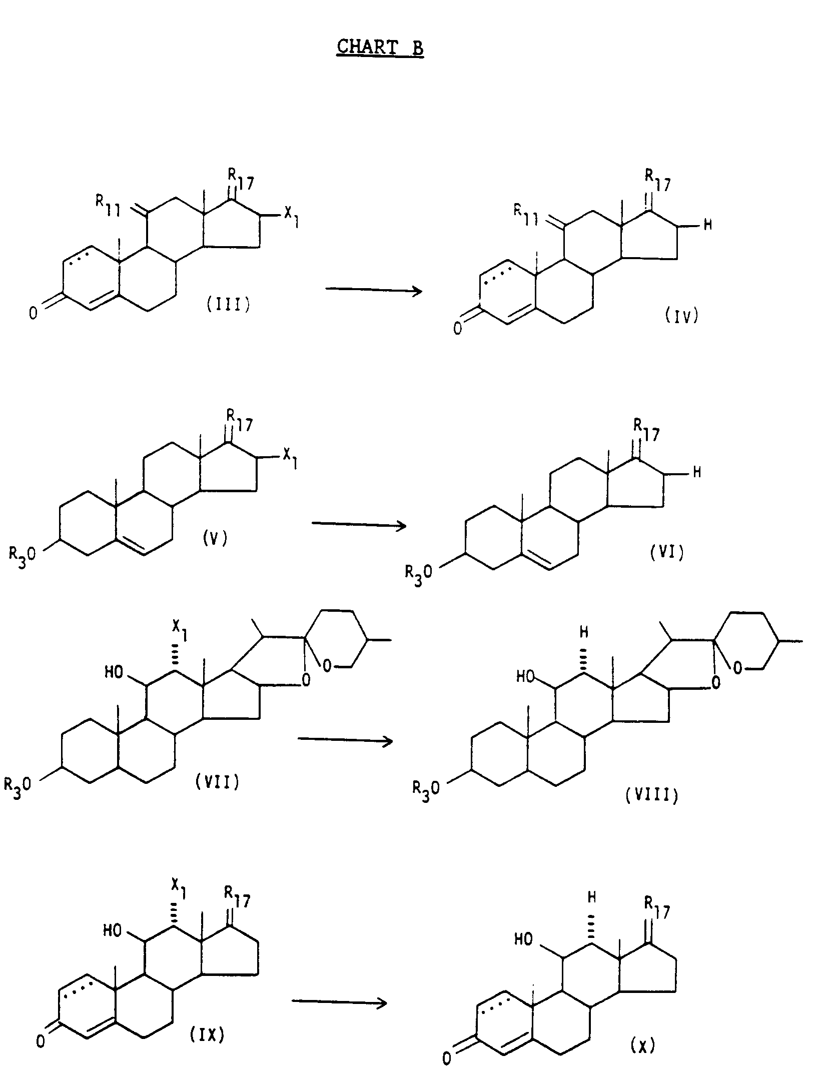

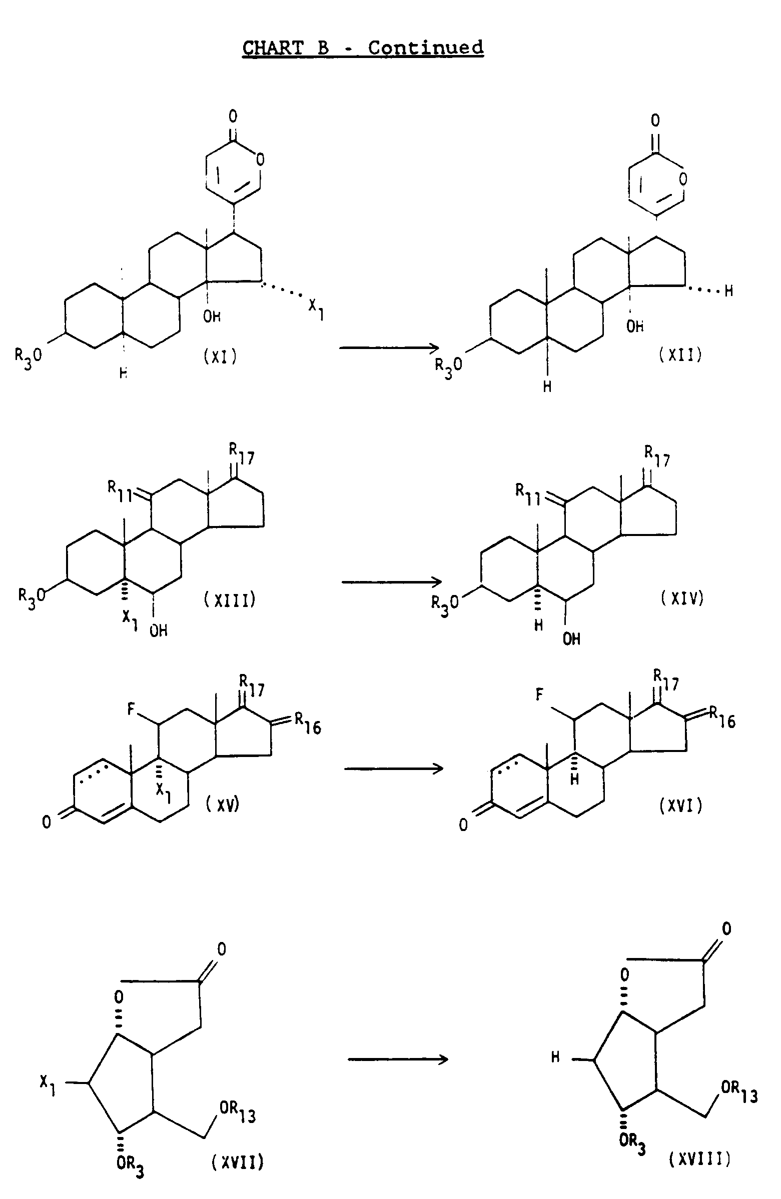

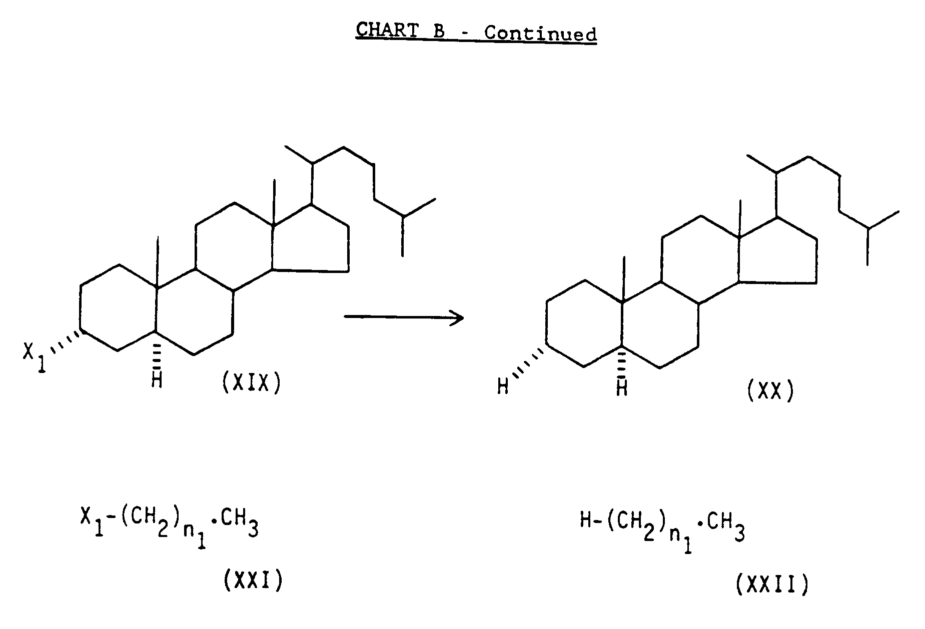

| CHART C | | |
|---|---|---|
| Initiator | Acceptable T | Preferred T |
| Oxygen (O₂) | 0 to 100° | 20 to 40° |
| Triethylborane optionally combine w/O₂ | -78 to 20° | -78 to -20° |
| Benzophenone and light of 250-350 mµ | -78 to 80° | 0 to 80° |
| Succinic acid peroxide | 40 to 100° | 60 to 90° |
| Dibenzoylperoxide | 40 to 110° | 80° |
| Lauroyl peroxide | 40 to 90° | 60 to 80° |
| Di-t-butylperoxyoxalate | 0 to 60° | 20° |
| t-Butylperbenzoate | 60 to 100° | 80 to 100° |
| Di-t-butylperoxide | 80 to 140° | 110 to 140° |
| Di-cumylperoxide | 100 to 140° | 120° |
| t-Butylhydroperoxide and acetic acid | 20 to 60° | 40 to 60° |
| t-Butylperbenzoate and cuprous bromide | 60 to 100° | 80° |
| H₂O₂ and ferrous perchlorate | 0 to 40° | 20° |
| Potassium persulfate | 40 to 80° | 60° |
| Potassium nitroso disulfonate | 40 to 80° | 60° |
| AIBN | 40 to 90° | 60° |
| 1,1'-Azobis(cyclohexanecarbonitrile) | 40 to 90° | 60° |
| Di-(2-ethylhexyl) peroxydicarbonate | 30 to 80° | 50° |
| t-Amyl peroxypivalate | 30 to 80° | 50° |
| Methylethylketone peroxide combined with cobalt naphthenate or cobalt octoate | 0 to 50° | 20° |

## Claims

1. A process for the preparation of an 11-hydroxy steroid of formula (II) 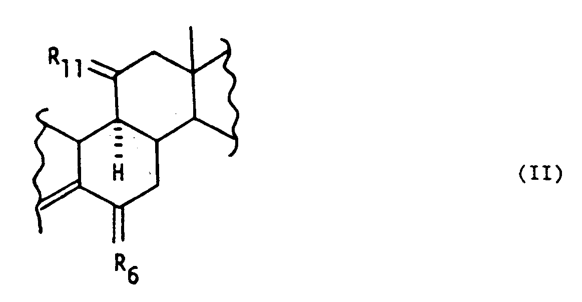 where R₆ is α-R₆₋₁:β-R₆₋₂, where one of R₆₋₁ and R₆₋₂ is H and the other is H, F or CH₃; and R₁₁ αH:βOH or αOH:β-H; which comprises contacting a corresponding 9α-halo steroid of formula (I) 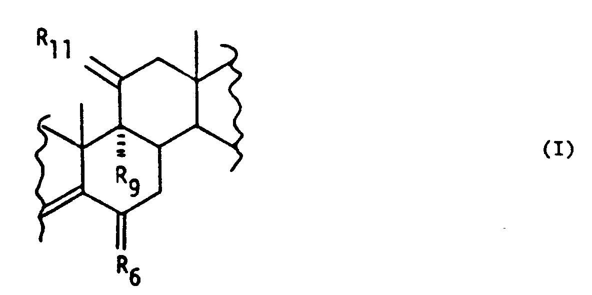 where R₉ is Cl, Br or I with a metal or salt selected from tin (O), tin (II) salts, lead (O) and lead (II) salts, a hydrogen atom donor and an initiator.

2. A process according to claim 1 where the 9α-halo steroid (I) is of the formula 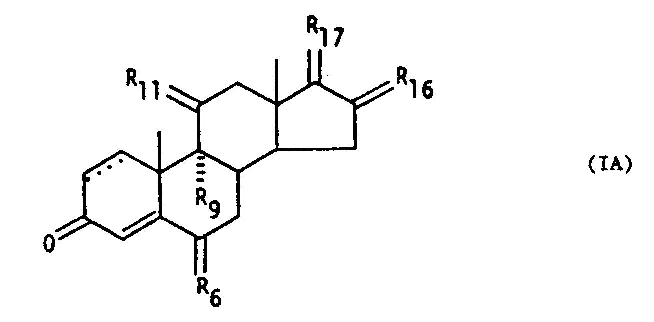 wherein R₆, R₉ and R₁₁ are as defined in claim 1;
.... is a single or double bond;
R₁₆ is α-R₁₆₋₁:β-R₁₆₋₂ where one of R₁₆₋₁ and R₁₆₋₂ is H and the other is H, OH or CH₃; and
R₁₇ is =O,
α-H:β-CO-CH₃,
α-OR₁₇₋₁:β-CO-CH₂-OR₂₁₋₁ where R₁₇₋₁ is H or -CO-R₁₇₋₂ where R₁₇₋₂ is C₁-C₃ alkyl or φ and R₂₁₋₁ is H or -CO-R₂₁₋₂ where R₂₁₋₂ is C₁-C₃ alkyl or φ optionally substituted with Cl or NO₂,
α-OR₁₇₋₃:β-CN where R₁₇₋₃ is
H,
THP,
-CH₂-OCH₃,
-CHR₁₇₋₃₁-O-R₁₇₋₃₂ where R₁₇₋₃₁ is C₁-C₃ alkyl and R₁₇₋₃₂ is C₁-C₄ alkyl or φ;
-SiR₁₇₋₃₃R₁₇₋₃₄R₁₇₋₃₅ where R₁₇₋₃₃, R₁₇₋₃₄ and R₁₇₋₃₅ are independently selected from C₁-C₄ alkyl, C₁-C₄ alkoxy, C₁-C₄ monohaloalkyl where halo is Br or Cl, and φ optionally substituted with 1 or 2 -OCH₃ or -NH₂; or
α-OR₁₇₋₄:β-CO-CH₃ where R₁₇₋₄ is H or -CO-R₁₇₋₄₁ where R₁₇₋₄₁ is C₂-C₄ alkyl or φ optionally substituted with 1 or 2 -OCH₃;
or the 16,17-acetonide thereof when R₁₆₋₁ is OH and R₁₇ is α-OH:β-CO-CH₂-OR₂₁₋₁.

3. A process according to claim 2 where .... is a double bond.

4. A process according to claim 3 where R₁₇ is α-OR₁₇₋₁:β-CO-CH₂-OR₂₁₋₁.

5. A process according to claim 2 where .... is a single bond.

6. A process according to claim 1 where the 11-oxygenated steroid (II) is prednisolone 21-acetate or hydrocortisone 21-acetate.

7. A process for the preparation of a compound selected from compounds of the formulae 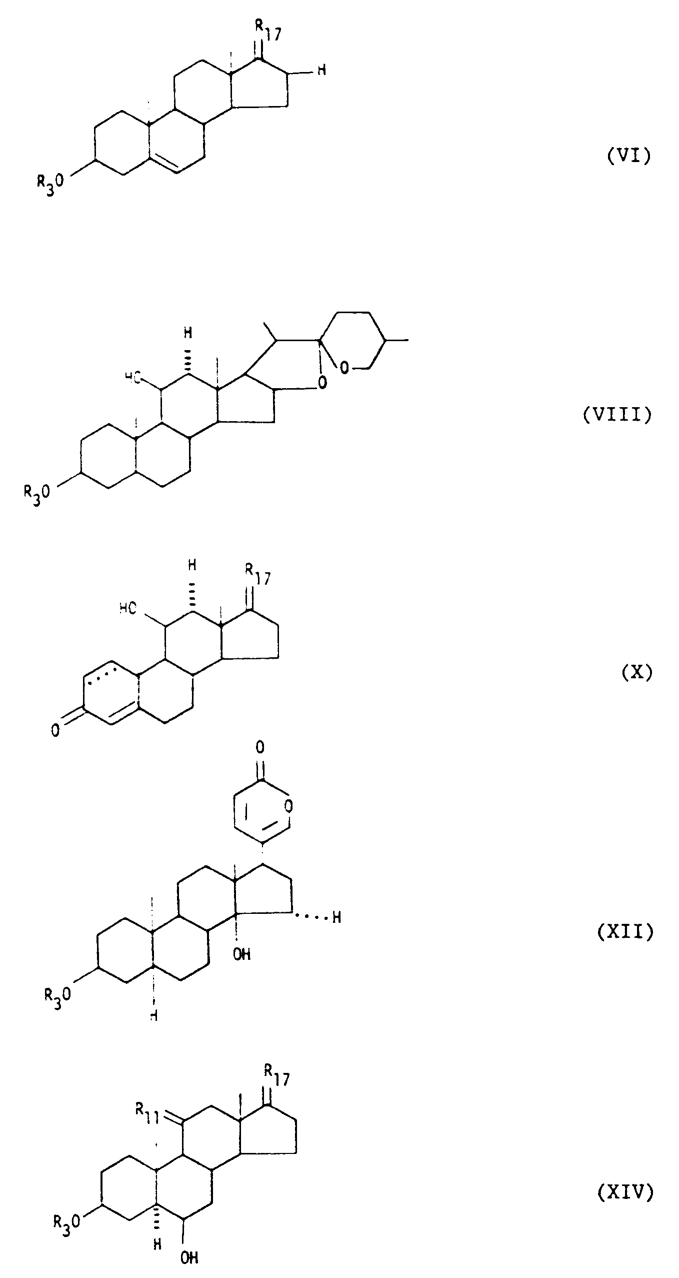 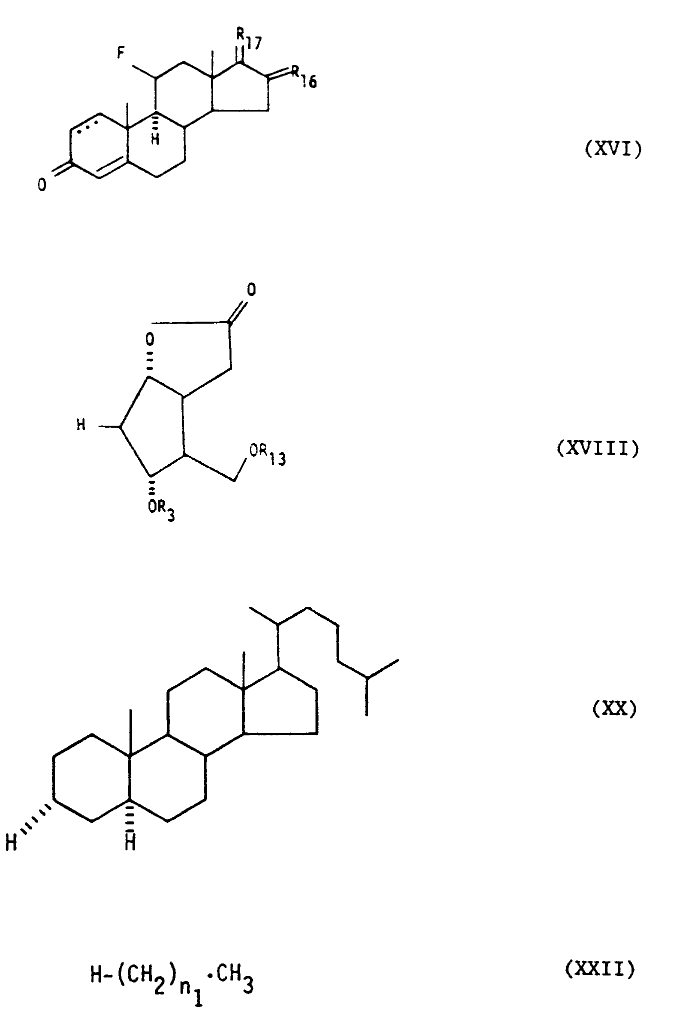 where R₃ is -H or -CO-R₃₋₁ where R₃₋₁ is C₁-C₃ alkyl or φ;
R₁₃ is H, C₁-C₄ alkyl or -CO-R₁₃₋₁ where R₁₃₋₁ is H, C₁₋₃ alkyl or φ;
n₁ is 9-20; and
...., R₁₁, R₁₆ and R₁₇ are as defined in claim 1 or claim 2, or the 16,17-acetonide thereof as defined in claim 2;
which comprises
(1) contacting the corresponding halogenated compound of the formula 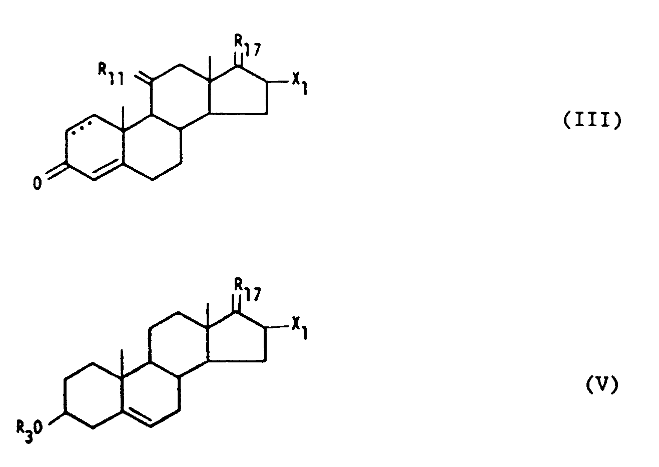 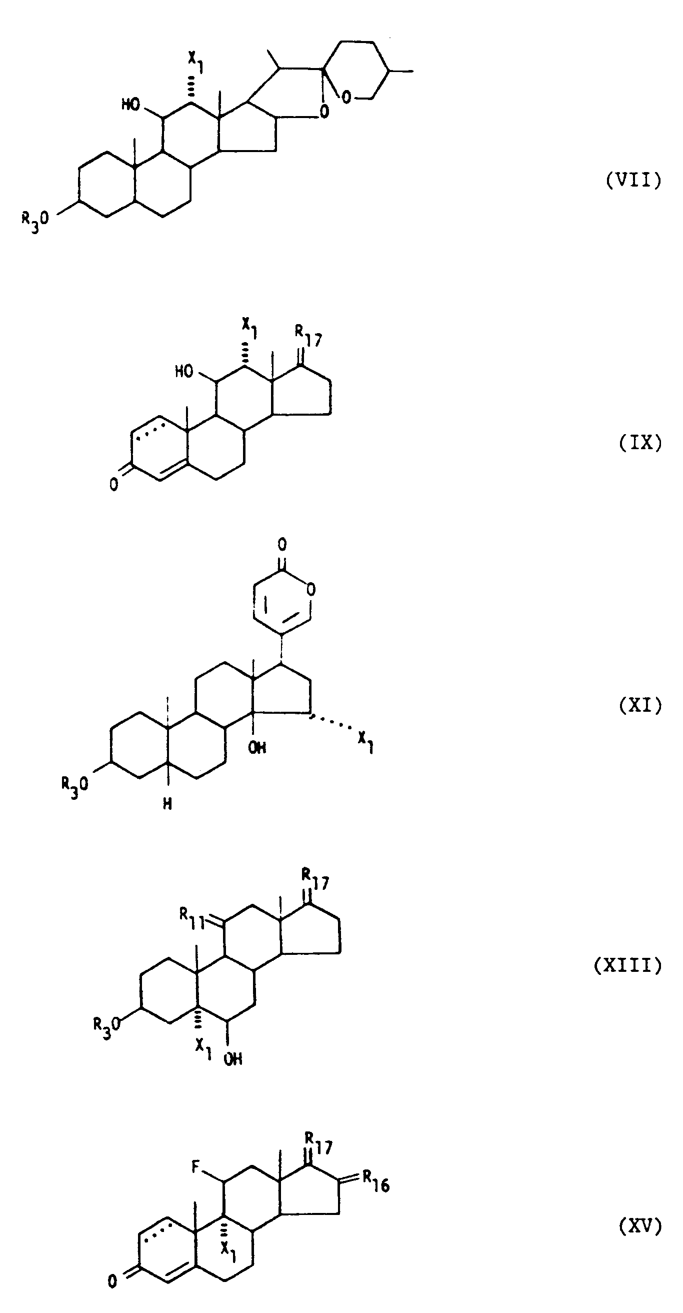 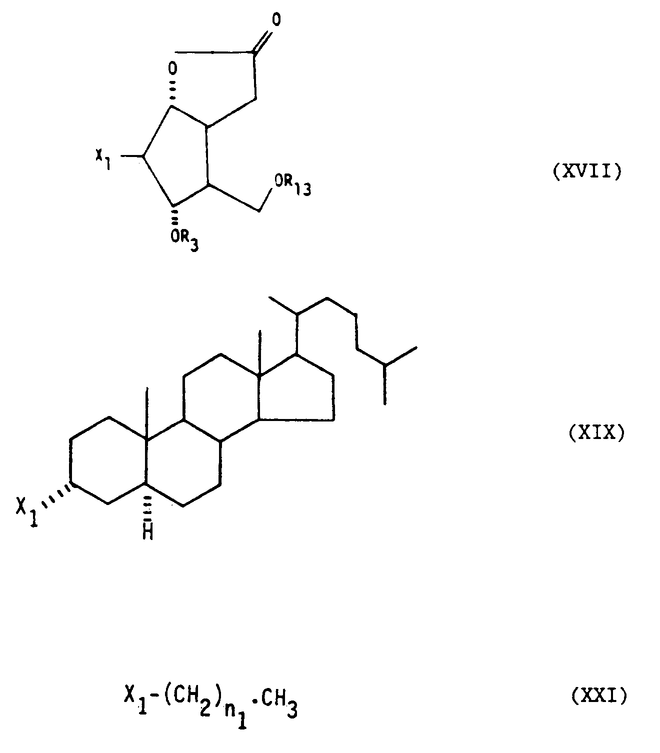 where X₁ is Cl, Br or I with a metal or salt selected from tin (O), tin (II) salts, lead (O) and lead (II) salts, a hydrogen atom donor and an initiator.

8. A process according to any preceding claim where the hydrogen atom donor is any compound which, when contacted with 9α-bromoprednisolone 21-acetate (I) and tin (O) in sec-butanol for 1 hour a 75 to 80°C produces prednisolone 21-acetate (II) in the same or higher yield than that which is obtained using hypophosphorous acid.

9. A process according to claim 8 where the hydrogen atom donor is selected from
hydrogen bromide,
hydrogen iodide,
hypophosphorous acid,
1,2- and 1,4-dihydrobenzene,
1,2- and 1,4-dihydrotoluene,
1,2- and 1,4-dihydro-(ortho, meta, or para)-xylene,
1,4-dihydronaphthalene,
9,10-dihydroanthracene,
cyclopentadiene,
1-benzyl-1,4-dihydronicotinamide,
3,5-di-t-butyl-4-hydroxytoluene,
H-Si-(X₂)₃, H-Sn-(X₂)₃, H-Ge-(X₂)₃, H-P-(X₂)₂, H-Se-X₂, H-B-(X₂)₃ or H-S-X₂ where, when more than one X₂ is present the X₂'s are the same or different and are -H,
C₁-C₁₀ alkyl,
C₅-C₁₀ cycloalkyl,
α-naphthyl,
β-naphthyl,
-φ optionally substituted with 1 or 2 X₃, where X₃ is
-F,
-Cl,
-Br,
-I,
-φ,
C₁-C₅ alkyl,
C₅-C₇ cycloalkyl,
-OX₄ where X₄ is -H, C₁-C₅ alkyl or C₅-C₇ cycloalkyl,
-COOX₄ where X₄ is as defined above,
-N(X₅)₂ where the X₅'s are the same or different and are -H, C₁-C₅, and where the X₅'s are taken together with the attached nitrogen atom and optionally another heteroatom, to form a heterocyclic ring selected from the group consisting of pyrrolidine, piperidine, morpholine, piperazine or N-methylpiperazine,
3-mercaptopropionic acid,
mercaptoacetic acid,
2-mercaptopropionic acid,
ethane dithiol,
propane 1,3-dithiol.

10. A process for the preparation of an 11-oxygenated steroid of formula (II) according to claim 9 where the hydrogen atom donor is H-S-X₂ where X₂ is as defined in claim 9.

11. A process for the preparation of an 11-oxygenated steroid of formula (II) according to claim 11 where the initiator is selected from the group consisting of AIBN, oxygen, triethylborane optionally combined with oxygen, 1,1'-azobis(cyclohexane carbonitrile), dibenzoylperoxide, lauroyl peroxide, succinic acid peroxide, di-t-butylperoxyoxalate, di(2-ethylhexyl)peroxydicarbonate t-butylperbenzoate, t-amylperoxypivalate, di-t-butylperoxide, dicumylperoxide, light of about 250 to about 350 mµ optionally combined with benzophenone, t-butylhydroperoxide optionally combined with acetic acid, hydrogen peroxide combined with ferrous perchlorate, t-butylperbenzoate combined with cuprous bromide, chloride or iodide, methylethylketone peroxide combined with cobalt naphthenate or cobalt octoate, potassium persulfate and potassium nitrosodisulfonate.

12. A process for the preparation of an 11-oxygenated steroid of formula (II) according to claim 11 where the initiator is AIBN or dibenzoylperoxide.

13. A process for the preparation of an 11-oxygenated steroid of formula (II) according to claim 1 where the contacting is performed in the presence of > 1 equivalent of a weak base defined as a base whose conjugate acid has a pKₐ of between about 2 and about 12.

14. A process for the preparation of an 11-oxygenated steroid of formula (II) according to claim 13 where the weak base is selected from the group consisting of
3-mercaptopropionate;
(X₆)₃-N where the X₆'s are the same or different and are
-H,
C₁-C₁₀ alkyl,
C₅-C₁₀ cycloalkyl,
α-naphthyl,
β-naphthyl,
-φ optionally substituted with 1 or 2 X₇, where X₇ is
-F,
-Cl,
-Br,
-I,
-φ,
C₁-C₅ alkyl,
C₅-C₇ cycloalkyl,
-OX₈ where X₈ is -H, C₁-C₅ alkyl or C₅-C₇ cycloalkyl,
-COOX₈ where X₈ is as defined above,
-N(X₉)₂ where the X₉'s are the same or different and are -H, C₁-C₅, and where the X₉'s are taken together with the attached nitrogen atom and optionally another heteroatom, to form a heterocyclic ring selected from the group consisting of pyrrolidine, piperidine, morpholine, piperazine or N-methylpiperazine,
X₆-COO⁻ where X₆ is as defined above;
citrate;
oxalate;
tartrate;
imidazole;
N-methylimidazole;
2-methylimidazole;
pyridine;
4-dimethylaminopyridine;
(any isomer of) lutidine;
collidine,
N,N'-dimethylpiperazine;
1,4-diazabicyclo[2.2.2]octane;
1,8-diazabicyclo[5.4.0]undec-7-ene or
1,5-diazabicyclo[4.3.0]non-5-ene.

15. A process according to any preceding claim where the metal or salt is tin (O) or a tin (II) salt.

## Patentansprüche

1. Verfahren zur Herstellung eines 11-Hydroxysteroids der Formel (II) 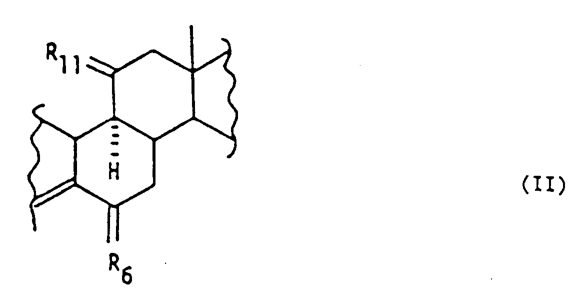 worin R₆ für α-R₆₋₁:β-R₆₋₂ mit einem der Reste R₆₋₁ und R₆₋₂ gleich H und dem anderen gleich H, F oder CH₃ steht und R₁₁ αH:βOH oder αOH:β-H darstellt, durch Inberührungbringen eines entsprechenden 9α-Halogensteroids der Formel (I) 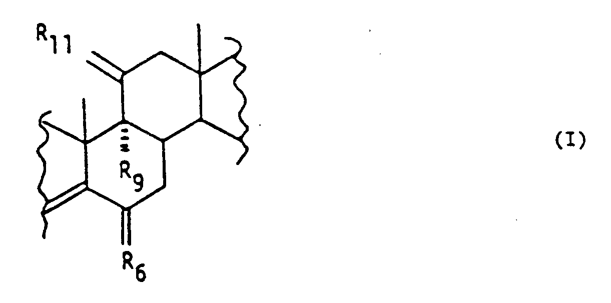 worin R₉ für Cl, Br oder I steht, mit einem Metall oder Salz, ausgewählt aus Zinn (0), Zinn (II)-Salzen, Blei (0) und Blei (II)-Salzen, einem Wasserstoffatomdonor und einem Anspringmittel.

2. Verfahren nach Anspruch 1, wobei das 9α-Halogensteroid (I) der Formel: 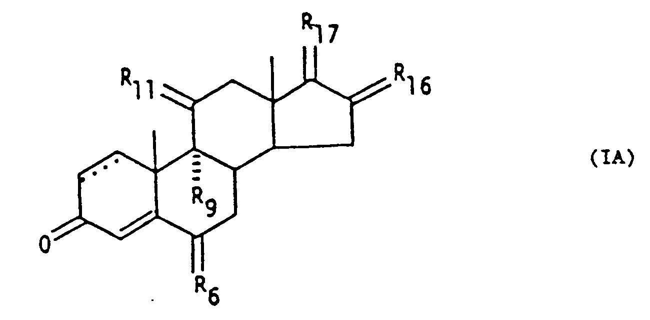 worin R₆, R₉ und R₁₁ die in Anspruch 1 angegebene Bedeutung besitzen, und worin ferner bedeuten:
.... eine Einfach- oder Doppelbindung;
R₁₆ α-R₁₆₋₁:β-R₁₆₋₂, worin einer der Reste R₁₆₋₁ und R₁₆₋₂ für H und der andere für H, OH oder CH₃ steht und
R₁₇ =O,
α-H:β-CO-CH₃,
α-OR₁₇₋₁:β-CO-CH₂-OR₂₁₋₁, worin R₁₇₋₁ für H oder
-CO-R₁₇₋₂ mit R₁₇₋₂ gleich C₁-C₃ Alkyl oder φ steht und R₂₁₋₁ H oder -CO-R₂₁₋₂ mit R₂₁₋₂ gleich C₁-C₃ Alkyl oder φ, gegebenenfalls substituiert durch Cl oder NO₂, darstellt,
α-OR₁₇₋₃:β-CN mit R₁₇₋₃ gleich
H,
THP,
-CH₂-OCH₃,
-CHR₁₇₋₃₁-O-R₁₇₋₃₂ mit R₁₇₋₃₁ gleich C₁-C₃ Alkyl und R₁₇₋₃₂ gleich C₁-C₄ Alkyl oder φ;
-SiR₁₇₋₃₃R₁₇₋₃₄R₁₇₋₃₅ mit R₁₇₋₃₃, R₁₇₋₃₄ und R₁₇₋₃₅ unabhängig voneinander ausgewählt aus C₁-C₄ Alkyl, C₁-C₄ Alkoxy, C₁-C₄ Monohalogenalkyl mit Br oder Cl als Halogen und φ, gegebenenfalls 1- oder 2fach substituiert durch -OCH₃ oder -NH₂; oder
α-OR₁₇₋₄:β-CO-CH₃ mit R₁₇₋₄ gleich H oder -CO-R₁₇₋₄₁,
worin R₁₇₋₄₁ für C₂-C₄ Alkyl oder φ, gegebenenfalls 1- oder 2fach substituiert durch -OCH₃, steht;
oder dem 16,17-Acetonid hiervon im Falle, daß R₁₆₋₁ für OH steht und R₁₇ α-OH:β-CO-CH₂-OR₂₁₋₁ darstellt.

3. Verfahren nach Anspruch 2, worin .... für eine Doppelbindung steht.

4. Verfahren nach Anspruch 3, worin R₁₇ α-OR₁₇₋₁:β-CO-CH₂-OR₂₁₋₁ darstellt.

5. Verfahren nach Anspruch 2, worin .... für eine Einfachbindung steht.

6. Verfahren nach Anspruch 1, wobei das 11-oxidierte Steroid (II) aus Prednisolon-21-acetat oder Hydrocortison-21-acetat besteht.

7. Verfahren zur Herstellung einer Verbindung, ausgewählt aus Verbindungen der Formeln: 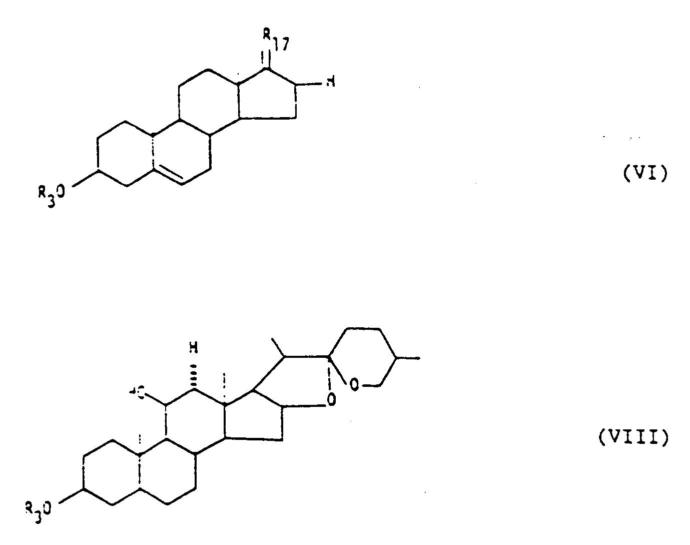 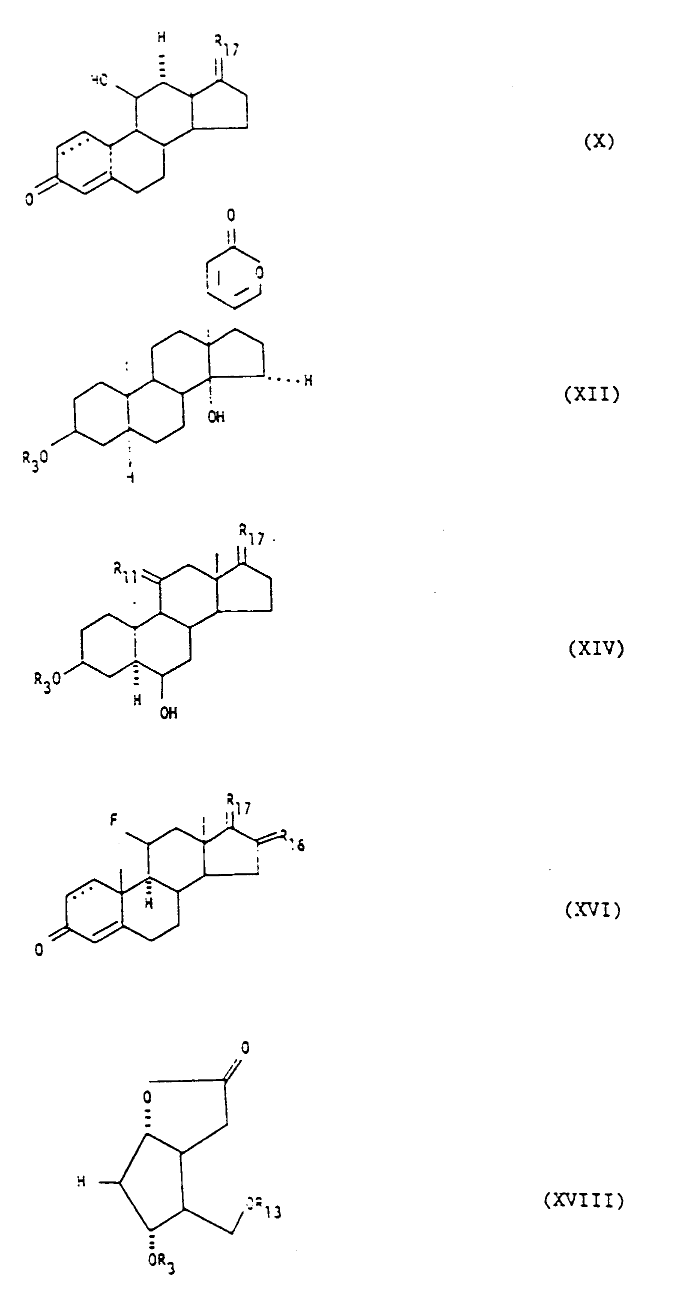 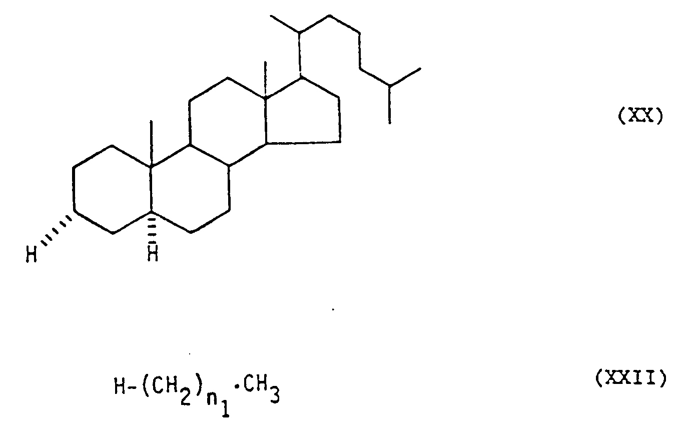 worin bedeuten:
R₃ -H oder -CO-R₃₋₁ mit R₃₋₁ gleich C₁-C₃ Alkyl oder φ;
R₁₃ H, C₁-C₄ Alkyl oder -CO-R₁₃₋₁ mit R₁₃₋₁ gleich H,
C₁-C₃ Alkyl oder φ;
n₁ = 9 - 20 und
worin .... R₁₁, R₁₆ und R₁₇ die in Anspruch 1 oder Anspruch 2 angegebene Bedeutung besitzen, oder des in Anspruch 2 definierten 16,17-Acetonids hiervon
durch
(1) Inberührungbringen der entsprechenden halogenierten Verbindung der Formel: 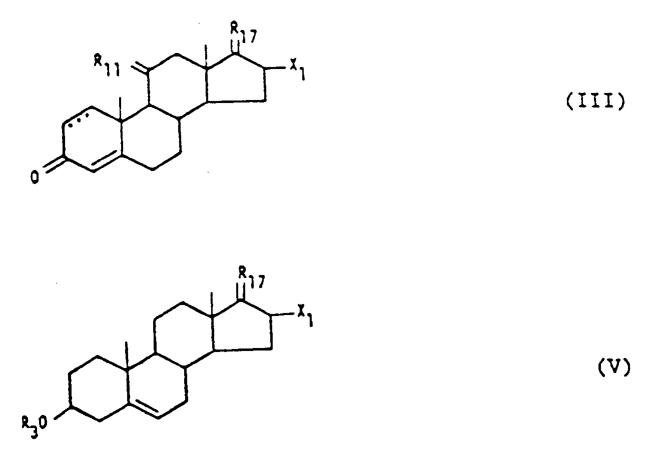 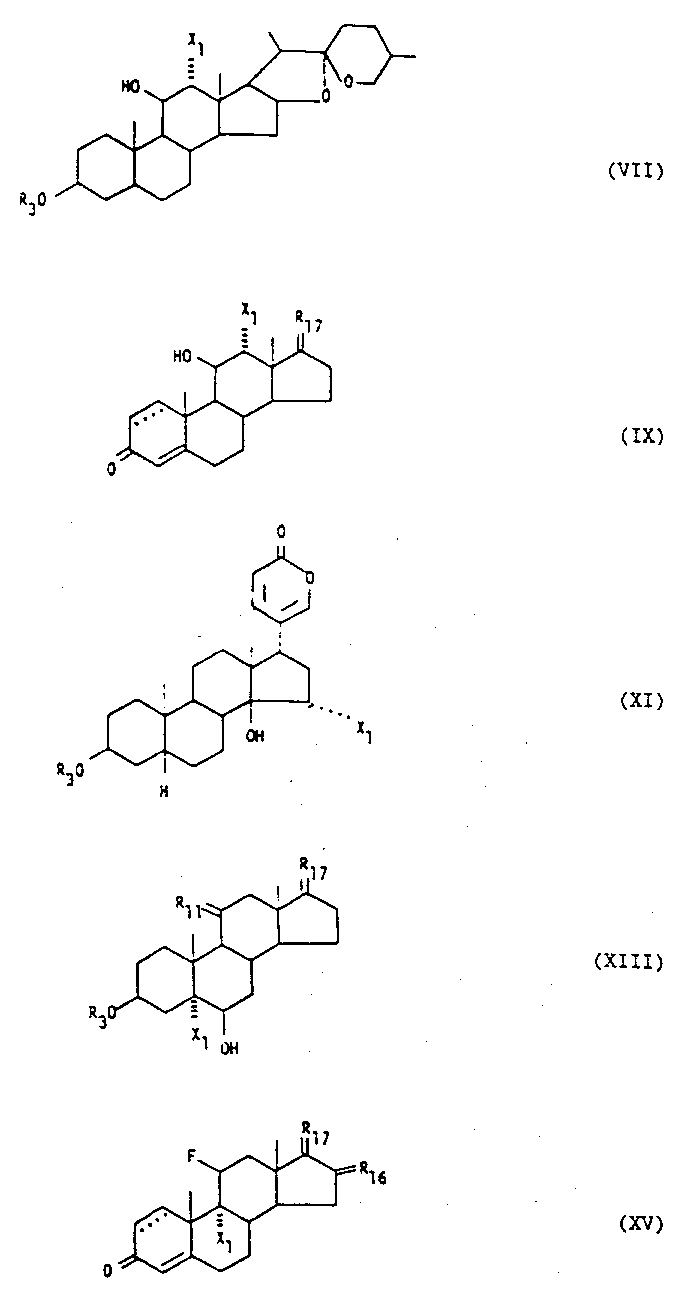 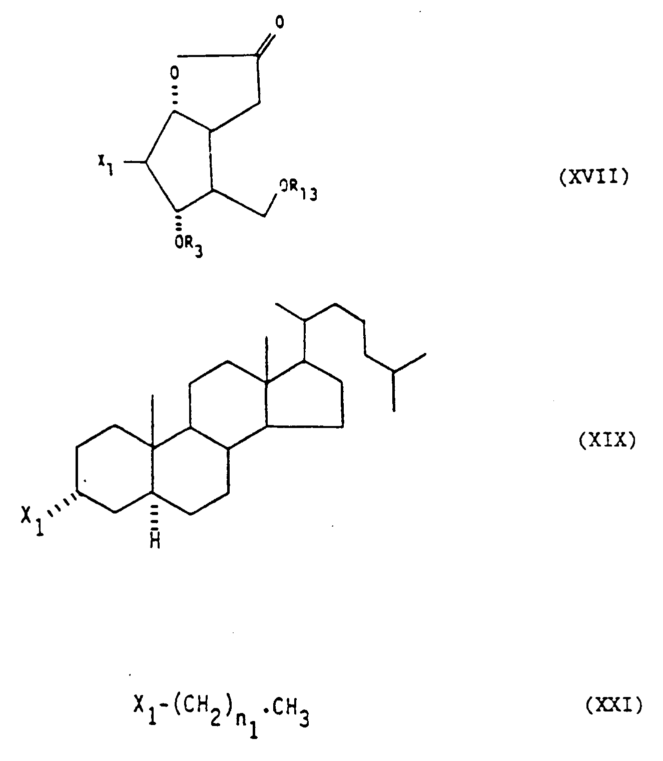 worin X₁ für Cl, Br oder I steht, mit einem Metall oder Salz, ausgewählt aus Zinn (0), Zinn (II)-Salzen, Blei (0) und Blei (II)-Salzen, einem Wasserstoffatomdonor und einem Anspringmittel.

8. Verfahren nach einem der vorhergehenden Ansprüche, wobei es sich bei dem Wasserstoffatomdonor um irgendeine Verbindung handelt, die beim Inberührungbringen mit 9α-Bromprednisolon 21-Acetat (I) und Zinn (0) in sek.-Butanol während 1 h bei 75 - 80°C Prednisolon 21-Acetat (II) in derselben oder höherer Ausbeute als bei Verwendung von hypophosphoriger Säure liefert.

9. Verfahren nach Anspruch 8, wobei der Wasserstoffatomdonor aus
Bromwasserstoff,
Jodwasserstoff,
hypophosphoriger Säure,
1,2- und 1,4-Dihydrobenzol,
1,2- und 1,4-Dihydrotoluol,
1,2- und 1,4-Dihydro-(ortho, meta oder para)-xylol,
1,4-Dihydronaphthalin,
9,10-Dihydroanthracen,
Cyclopentadien,
1-Benzyl-1,4-dihydronicotinamid,
3,5-Di-tert.-butyl-4-hydroxytoluol,
H-Si-(X₂)₃, H-Sn-(X₂)₃, H-Ge-(X₂)₃, H-P-(X₂)₂, H-Se-X₂, H-B-(X₂)₃ oder H-S-X₂, wobei bei Anwesenheit von mehr als einem Rest X₂ die Reste X₂ gleich oder verschieden sind und für -H,
C₁-C₁₀ Alkyl,
C₅-C₁₀ Cycloalkyl,
α-Naphthyl,
β-Naphthyl,
-φ, gegebenenfalls 1- oder 2fach substituiert
durch X₃, welches aus
-F,
-Cl,
-Br,
-I,
-φ,
C₁-C₅ Alkyl,
C₅-C₇ Cycloalkyl,
-OX₄ mit X₄ gleich -H, C₁-C₅ Alkyl oder C₅-C₇ Cycloalkyl,
-COOX₄ mit X₄ in der zuvor angegebenen Bedeutung,
-N(X₅)₂, worin die Reste X₅ gleich oder verschieden sind und -H, C₁-C₅ bedeuten und worin die Reste X₅ zusammen mit dem Stickstoffatom, an dem sie hängen, und gegebenenfalls einem weiteren Heteroatom einen heterocyclischen Ring, ausgewählt aus der Gruppe Pyrrolidin, Piperidin, Morpholin, Piperazin oder N-Methylpiperazin, bilden, besteht, steht,
3-Mercaptopropionsäure,
Mercaptoessigsäure,
2-Mercaptopropionsäure,
Ethandithiol,
Propan-1,3-dithiol
ausgewählt ist.

10. Verfahren zur Herstellung eines 11-oxidierten Steroids der Formel (II) nach Anspruch 9, wobei der Wasserstoffatomdonor aus H-S-X₂ mit X₂ in der in Anspruch 9 angegebenen Bedeutung besteht.

11. Verfahren zur Herstellung eines 11-oxidierten Steroids der Formel (II) nach Anspruch 11, wobei das Anspringmittel aus der Gruppe AIBN, Sauerstoff, Triethylboran, gegebenenfalls kombiniert mit Sauerstoff, 1,1'-Azo- bis (cyclohexancarbonitril), Dibenzoylperoxid, Lauroylperoxid, Bernsteinsäureperoxid, Di-tert.-butylperoxyoxalat, Di(2-ethylhexyl)peroxydicarbonat, tert.-Butylperbenzoat, tert.-Amylperoxypivalat, Di-tert.-butylperoxid, Dicumylperoxid, Licht einer Wellenlänge von etwa 250 bis etwa 350 mµ, gegebenenfalls in Kombination mit Benzophenon, tert.-Butylhydroperoxid, gegebenenfalls in Kombination mit Essigsäure, Wasserstoffperoxid in Kombination mit Eisen(II)-perchlorat, tert.-Butylperbenzoat in Kombination mit Kupfer(I)-bromid, -chlorid oder -jodid, Methylethylketonperoxid in Kombination mit Kobaltnaphthenat oder Kobaltoctoat, Kaliumpersulfat und Kaliumnitrosodisulfonat ausgewählt ist.

12. Verfahren zur Herstellung eines 11-oxydierten Steroids der Formel (II) nach Anspruch 11, wobei das Anspringmittel aus AIBN oder Dibenzoylperoxid besteht.

13. Verfahren zur Herstellung eines 11-oxydierten Steroids der Formel (II) nach Anspruch 1, wobei das Inberührungbringen in Gegenwart von > 1 Äquivalent einer schwachen Base (definiert als Base, deren Konjugatsäure einen pKₐ-Wert zwischen etwa 2 und etwa 12 aufweist) erfolgt.

14. Verfahren zur Herstellung eines 11-oxydierten Steroids der Formel (II) nach Anspruch 13, wobei die schwache Base aus der Gruppe
3-Mercaptopropionat;
(X₆)₃-N, worin die Reste X₆ gleich oder verschieden sind und für
-H,
C₁-C₁₀ Alkyl,
C₅-C₁₀ Cycloalkyl,
α-Naphthyl,
β-Naphthyl,
-φ, gegebenenfalls 1- oder 2fach durch X₇ substituiert, wobei X₇ für
-F,
-Cl,
-Br,
-I,
-φ,
C₁-C₅ Alkyl,
C₅-C₇ Cycloalkyl,
-OX₈ mit X₈ gleich -H, C₁-C₅ Alkyl oder
C₅-C₇ Cycloalkyl,
-COOX₈ mit X₈ in der zuvor angegebenen Bedeutung,
-N(X₉)₂, worin die Reste X₉ gleich oder verschieden sind und für -H, C₁-C₅ stehen und wobei die Reste X₉ zusammen mit dem Stickstoffatom, an dem sie hängen, und gegebenenfalls einem weiteren Heteroatom einen heterocyclischen Ring, ausgewählt aus der Gruppe Pyrrolidin, Piperidin, Morpholin, Piperazin oder N-Methylpiperazin, bilden, steht, steht,
X₆-COO⁻ mit X₆ in der zuvor angegebenen Bedeutung;
Citrat;
Oxalat;
Tartrat,
Imidazol;
N-Methylimidazol;
2-Methylimidazol;
Pyridin;
4-Dimethylaminopyridin;
(jedes Isomere von) Lutidin;
Collidin,
N,N'-Dimethylpiperazin;
1,4-Diazabicyclo[2.2.2]octan;
1,8-Diazabicyclo[5.4.0]undec-7-en oder
1,5-Diazabicyclo[4.3.0]non-5-en
ausgewählt ist.

15. Verfahren nach einem der vorhergehenden Ansprüche, wobei das Metall oder Salz aus Zinn (0) oder einem Zinn (II)-Salz besteht.

## Revendications

1. Procédé de préparation d'un 11-hydroxystéroïde de formule (II) 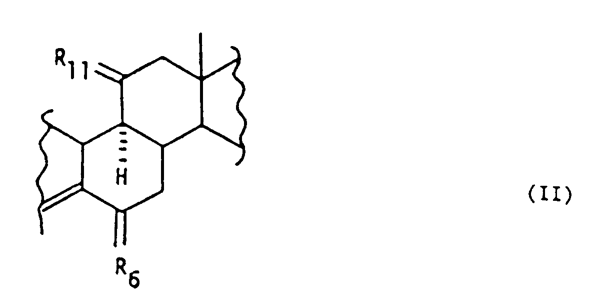 dans laquelle R₆ représente un groupe α-R₆₋₁:β-R₆₋₂, dans lequel un des groupes R₆₋₁ et R₆₋₂ représente H et l'autre représente H, F ou un groupe CH₃ ; et R₁₁ représente un groupe αH:βOH ou αOH:βH, qui comprend la mise en contact d'un 9α-halogénostéroïde correspondant de formule (I) 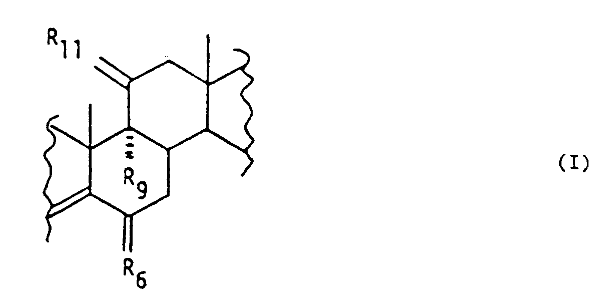 dans laquelle R₉ représente Cl, Br ou I avec un métal ou un sel choisi entre l'étain (0), des sels d'étain (II), le plomb (0) et des sels de plomb (II), un donneur d'atomes d'hydrogène et un initiateur.

2. Procédé suivant la revendication 1, dans lequel le 9α-halogénostéroïde (I) répond à la formule 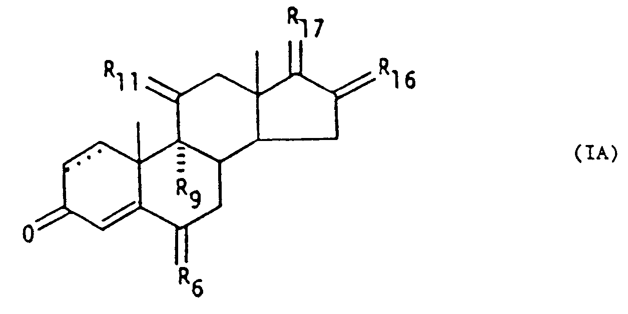 dans laquelle R₆, R₉ et R₁₁ répondent aux définitions suivant la revendication 1 ;
.... représente une liaison simple ou une double liaison ;
R₁₆ représente un groupe α-R₁₆₋₁:β-R₁₆₋₂ dans lequel un des groupes R₁₆₋₁ et R₁₆₋₂ représente H et l'autre représente H, un groupe OH ou CH₃ ; et
R₁₇ représente =0,
un groupe α-H:β-CO-CH₃,
un groupe α-OR₁₇₋₁:β-CO-CH₂-OR₂₁₋₁ dans lequel R₁₇₋₁ représente H ou un groupe -CO-R₁₇₋₂ dans lequel R₁₇₋₂ représente un groupe alkyle en C₁ à C₃ ou φ et R₂₁₋₁ représente H ou un groupe -CO-R₂₁₋₂ dans lequel R₂₁₋₂ représente un groupe alkyle en C₁ à C₃ ou φ facultativement substitué avec Cl ou un groupe NO₂,
un groupe α-OR₁₇₋₃:β-CN dans lequel R₁₇₋₃ représente
H,
un groupe THP,
un groupe -CH₂-OCH₃,
un groupe -CHR₁₇₋₃₁-O-R₁₇₋₃₂ dans lequel R₁₇₋₃₁ représente un groupe alkyle en C₁ à C₃ et R₁₇₋₃₂ représente un groupe alkyle en C₁ à C₄ ou φ ;
un groupe -SiR₁₇₋₃₃R₁₇₋₃₄R₁₇₋₃₅ dans lequel R₁₇₋₃₃, R₁₇₋₃₄ et R₁₇₋₃₅ sont choisis indépendamment entre des groupes alkyle en C₁ à C₄, alkoxy en C₁ à C₄, monohalogénalkyle en C₁ à C₄, halogéno représentant Br ou Cl, et φ facultativement substitué avec 1 ou 2 groupes -OCH₃ ou -NH₂8 ; ou
un groupe α-OR₁₇₋₄:β-CO-CH₃ dans lequel R₁₇₋₄ représente H ou un groupe -CO-R₁₇₋₄₁ dans lequel R₁₇₋₄₁ représente un groupe alkyle en C₂ à C₄ ou φ facultativement substitué avec 1 ou 2 groupes -OCH₃ ;
ou son 16,17-acétonide lorsque R₁₆₋₁ représente un groupe OH et R₁₇ représente un groupe α-OH:β-CO-CH₂-OR₂₁₋₁.

3. Procédé suivant la revendication 2, dans lequel .... représente une double liaison.

4. Procédé suivant la revendication 3, dans lequel R₁₇ représente un groupe α-OR₁₇₋₁:β-CO-CH₂-OR₂₁₋₁.

5. Procédé suivant la revendication 2, dans lequel .... représente une liaison simple.

6. Procédé suivant la revendication 1, dans lequel le stéroïde oxygéné en position 11 (II) est le 21-acétate de prednisolone ou le 21-acétate d'hydrocortisone.

7. Procédé de préparation d'un composé choisi entre des composés de formules 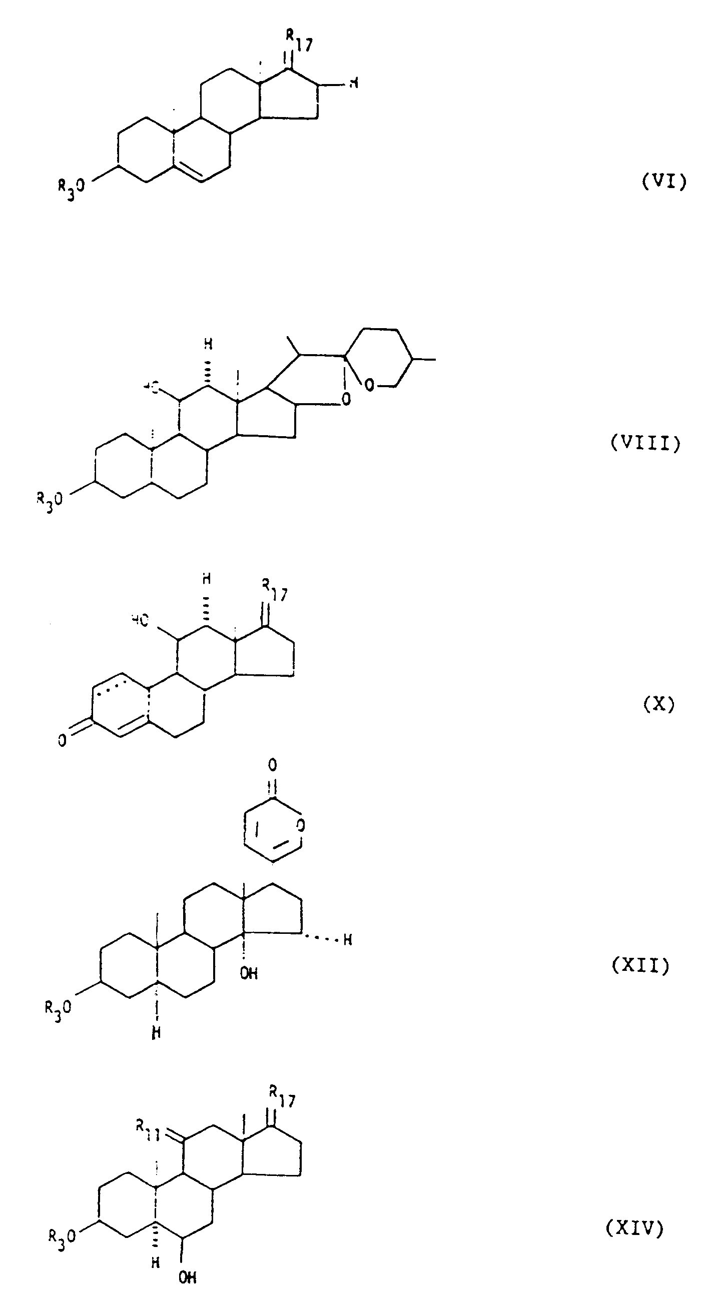 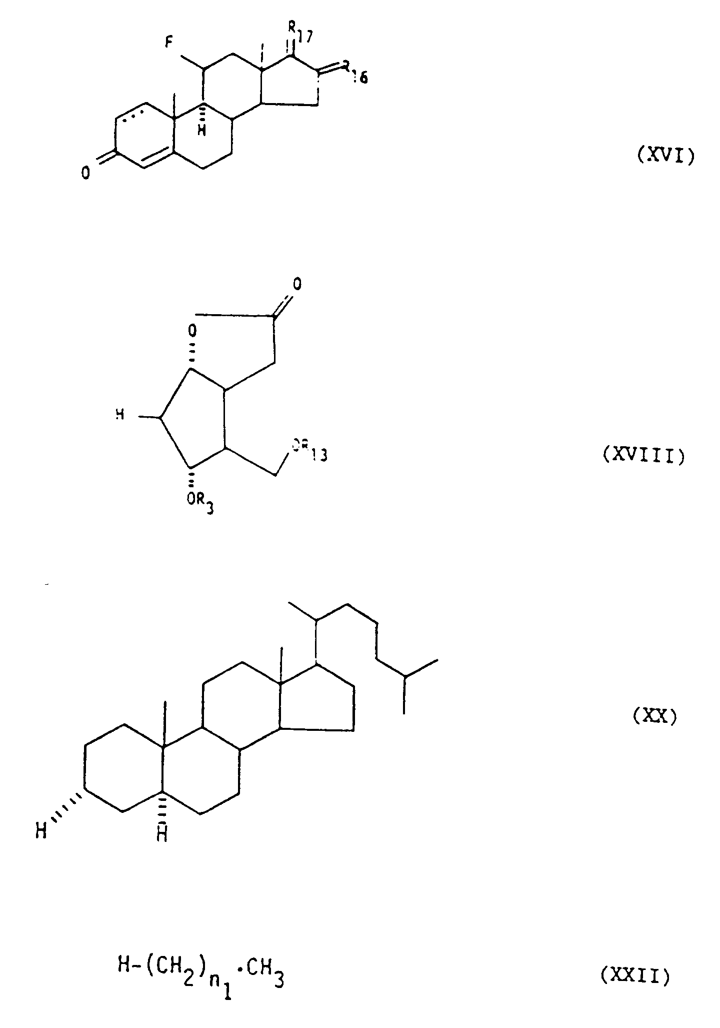 dans lesquelles R₃ représente -H ou un groupe -CO-R₃₋₁ dans lequel R₃₋₁ représente un groupe alkyle en C₁ à C₃ ou φ ;
R₁₃ représente H, un groupe alkyle en C₁ à C₄ ou -CO-R₁₃₋₁ dans lequel R₁₃₋₁ représente H, un groupe alkyle en C₁ à C₃ ou φ ;
n₁ à une valeur de 9 à 20 ; et
...., R₁₁, R₁₆ et R₁₇ répondent aux définitions suivant la revendication 1 ou la revendication 2, ou de son 16,17-acétonide tel que défini dans la revendication 2 ;
qui comprend
(1) la mise en contact du composé halogéné correspondant, de formule 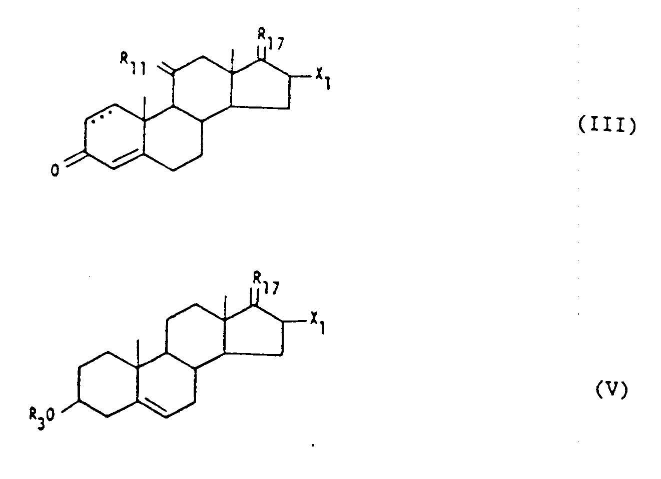 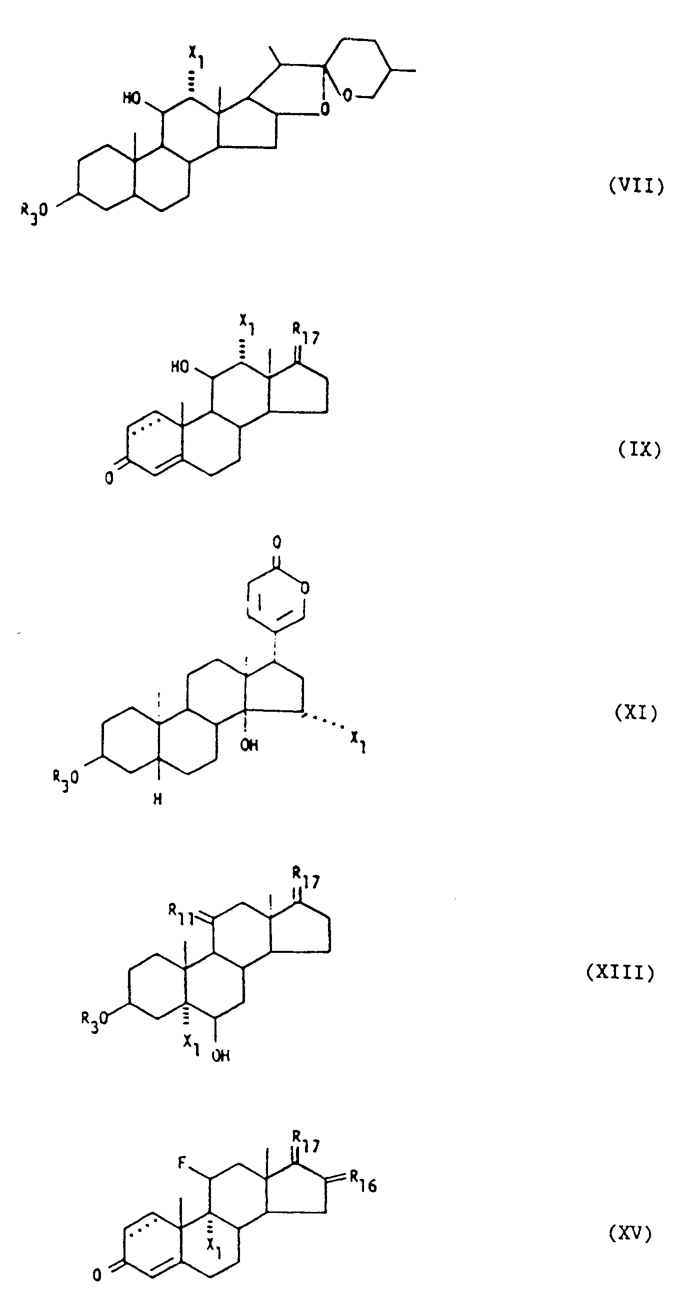 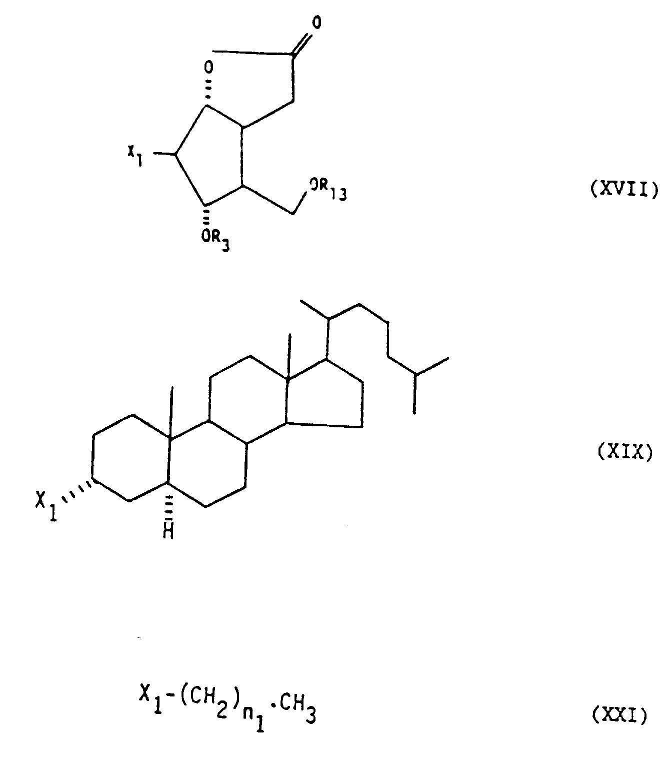 dans laquelle X₁ représente Cl, Br ou I, avec un métal ou un sel choisi entre l'étain (0), des sels d'étain (II), le plomb (0) et des sels de plomb (II), un donneur d'atomes d'hydrogène et un initiateur.

8. Procédé suivant l'une quelconque des revendications précédentes, dans lequel le donneur d'atomes d'hydrogène est n'importe quel composé qui, lors de sa mise en contact avec le 21-acétate de 9α-bromoprednisolone (I) et l'étain (0) dans le sec.-butanol pendant 1 heure à une température de 75 à 80°C produit du 21-acétate de prednisolone (II) en un rendement identique ou supérieur à celui qui est obtenu au moyen d'acide hypophosphoreux.

9. Procédé suivant la revendication 8, dans lequel le donneur d'atomes d'hydrogène est choisi entre
l'acide bromhydrique,
l'acide iodhydrique,
l'acide hypophosphoreux,
les 1,2- et 1,4-dihydrobenzènes,
les 1,2- et 1,4-dihydrotoluènes,
les 1,2- et 1,4-dihydro-(ortho, méta ou para)-xylènes,
le 1,4-dihydronaphtalène,
le 9,10-dihydroanthracène,
le cyclopentadiène,
le 1-benzyl-1,4-dihydronicotinamide,
le 3,5-di-tertiobutyl-4-hydroxytoluène,
H-Si-(X₂)₃, H-Sn-(X₂)₃_{,} H-Ge-(X₂)₃_{,} H-P-(X₂)₂, H-Se-X₂, H-B-(X₂)₃ ou H-S-X₂, dans lequel, lorsque plus d'un groupe X₂ est présent, les groupes X₂ sont identiques ou différents et représentent -H,
des groupes alkyle en C₁ à C₁₀,
des groupes cycloalkyle en C₅ à C₁₀,
des groupes α-naphtyle,
des groupes β-naphtyle,
des groupes φ, facultativement substitués
avec 1 ou 2 groupes X₃, X₃ représentant
-F,
-Cl,
-Br,
-I,
un groupe φ,
un groupe alkyle en C₁ à C₅,
un groupe cycloalkyle en C₅ à C₇,
un groupe OX₄ dans lequel X₄ représente
-H, un groupe alkyle en C₁ à C₅ ou cycloalkyle en C₅ à C₇,
un groupe COOX₄ dans lequel X₄ répond à la définition précitée,
un groupe -N(X₅)₂ dans lequel les groupes X₅ sont identiques ou différents et représentent -H ou des groupes alkyle en C₁ à C₅, ou bien dans lequel les groupes X₅ sont pris conjointement avec l'atome d'azote auquel ils sont fixés et facultativement avec un autre hétéroatome en formant un noyau hétérocyclique choisi dans le groupe consistant en la pyrrolidine, la pipéridine, la morpholine, la pipérazine ou la N-méthylpipérazine,
l'acide 3-mercaptopropionique,
l'acide mercaptoacétique,
l'acide 2-mercaptopropionique,
l'éthane-dithiol,
le propane-1,3-dithiol.

10. Procédé de préparation d'un stéroïde oxygéné en position 11, de formule (II), suivant la revendication 9, dans lequel le donneur d'atomes d'hydrogène est un composé de formule H-S-X₂ dans laquelle X₂ répond à la définition suivant la revendication 9.

11. Procédé de préparation d'un stéroïde oxygéné en position 11, de formule (II), suivant la revendication 8, dans lequel l'initiateur est choisi dans le groupe consistant en AIBN, oxygène, triéthylborane facultativement associé à l'oxygène, 1,1'-azobis(cyclohexanecarbonitrile), peroxyde de dibenzoyle, peroxyde de lauroyle, peroxyde d'acide succinique, peroxyoxalate de di-tertiobutyle, peroxydicarbonate de di-(2-éthylhexyle), perbenzoate de tertiobutyle, peroxypivalate de tertio-amyle, peroxyde de di-tertiobutyle, peroxyde de dicumyle, lumière d'environ 250 à environ 350 nm facultativement en association avec la benzophénone, hydroperoxyde de tertiobutyle facultativement en association avec l'acide acétique, peroxyde d'hydrogène en association avec le perchlorate ferreux, perbenzoate de tertiobutyle en association avec le bromure, chlorure ou iodure cuivreux, peroxyde de méthyléthylcétone en association avec le naphténate de cobalt ou l'octoate de cobalt, persulfate de potassium et nitrosodisulfonate de potassium.

12. Procédé de préparation d'un stéroïde oxygéné en position 11, de formule (II), suivant la revendication 11, dans lequel l'initiateur est le AIBN ou le peroxyde de dibenzoyle.

13. Procédé de préparation d'un stéroïde oxygéné en position 11, de formule (II), suivant la revendication 1, dans lequel la mise en contact est effectuée en présence de plus d'un équivalent d'une base faible définie comme étant une base dont l'acide conjugué possède un PKₐ compris dans l'intervalle d'environ 2 à environ 12.

14. Procédé de préparation d'un stéroïde oxygéné en position 11, de formule (II), suivant la revendication 13, dans lequel la base faible est choisie dans le groupe consistant en
un 3-mercaptopropionate ;
un composé de formule (X₆)₃-N dans laquelle les groupes X₆ sont identiques ou différents et représentent
-H,
des groupes alkyle en C₁ à C₁₀,
des groupes cycloalkyle en C₅ à C₁₀,
des groupes α-naphtyle,
des groupes β-naphtyle,
des groupes -φ, facultativement substitués avec 1 ou 2 groupes X₇, X₇ représentant
-F,
-Cl,
-Br,
-I,
des groupes -φ,
des groupes alkyle en C₁ à C₅,
des groupes cycloalkyle en C₅ à C₇,
des groupes -OX₈ dans lesquels X₈ re
présente -H, un groupe alkyle en C₁ à C₅
ou cycloalkyle en C₅ à C₇,
des groupes -COOX₈ dans lesquels X₈ ré
pond à la définition précitée,
des groupes -N(X₉)₂ dans lesquels les groupes X₉ sont identiques ou différents et représentent -H ou des groupes alkyle en C₁ à C₅, ou bien dans lesquels les groupes X₉ sont pris conjointement avec l'atome d'azote auquel ils sont fixés et facultativement avec un autre hétéroatome en formant un noyau hétérocyclique choisi dans le groupe consistant en la pyrrolidine, la pipéridine, la morpholine, la pipérazine et la N-méthylpipérazine,
un groupe X₆-COO⁻ dans lequel X₆ répond à la définition précitée ;
un citrate ;
un oxalate ;
un tartrate ;
l'imidazole ;
le N-méthylimidazole ;
le 2-méthylimidazole ;
la pyridine ;
la 4-diméthylaminopyridine ;
(n'importe quel isomère de) la lutidine ;
la collidine ;
la N,N'-diméthylpipérazine ;
le 1,4-diazabicyclo[2.2.2]octane ;
le 1,8-diazabicyclo[5.4.0]undéc-7-ène, ou
le 1,5-diazabicyclo[4.3.0]non-5-ène.

15. Procédé suivant l'une quelconque des revendications précédentes, dans lequel le métal ou sel est l'étain (0) ou un sel d'étain (II).
